# EUROPEAN PATENT SPECIFICATION

(11) **EP 4 487 893 B1**
(45) Date of publication and mention of the grant of the patent: **22.07.2026**
(21) Application number: 24194015.4
(22) Date of filing: 27.05.2015
(51) Int. Cl.: G16H 40/67, G16H 20/40, A61M 16/00, A61M 16/06, A61M 16/08, A61M 16/10, A61M 16/16, A61B 5/087

(54) **REMOTE DATA MANAGEMENT FOR MEDICAL DEVICES**
FERNDATENVERWALTUNG FÜR MEDIZINISCHE VORRICHTUNGEN
GESTION DE DONNÉES À DISTANCE POUR DISPOSITIFS MÉDICAUX

(30) Priority: 27.05.2014 AU 2014901998
(43) Date of publication of application: 08.01.2025
(62) Divisional of application: 15799348.6
(73) Proprietor: ResMed, Inc., San Diego CA 92123 (US)
(72) Inventor: DELANGRE, Peter, Bella Vista, New South Wales 2153 (AU); BIRCHALL, Paul Frederick, Bella Vista, New South Wales 2153 (AU); CHURCHILL, Dawn Rosemary, Bella Vista, New South Wales 2153 (AU); PRICE, Jason Stelakis, Hornsby, New South Wales 2077 (AU); ROBERTS, Christopher John, San Diego, CA 92123 (US); SOMAIYA, Chinmayee, Bella Vista, New South Wales 2153 (AU); TEMPLETON, Bradley Scott, Abingdon Oxfordshire OX14 4RX (GB); TRULL, Wendall Eric, San Diego, CA 92123 (US); TYLER, Matthew Scott, San Diego, CA 92123 (US)
(74) Representative: Vossius & Partner Patentanwälte Rechtsanwälte mbB

(56) References cited:
- EP-A1- 1 900 387
- WO-A1-2013/002650
- US-A1- 2003 236 450
- US-A1- 2008 251 078
- US-A1- 2013 269 700
- US-A1- 2014 032 231

## Description

### CROSS-REFERENCES TO RELATED APPLICATIONS

The present application claims priority to Australian Provisional Application No. 2014901998, filed on May 27, 2014.

### 1 BACKGROUND

### 1.1 (1) FIELD OF THE TECHNOLOGY

The present technology relates to one or more of the detection, diagnosis, treatment, prevention and amelioration of respiratory-related disorders. In particular, the present technology relates to medical devices or apparatus, and their use.

### 1.2 (2) DESCRIPTION OF THE RELATED ART

### 1.2.1 Human Respiratory System and its Disorders

The respiratory system of the body facilitates gas exchange. The nose and mouth form the entrance to the airways of a patient.

The airways include a series of branching tubes, which become narrower, shorter and more numerous as they penetrate deeper into the lung. The prime function of the lung is gas exchange, allowing oxygen to move from the air into the venous blood and carbon dioxide to move out. The trachea divides into right and left main bronchi, which further divide eventually into terminal bronchioles. The bronchi make up the conducting airways, and do not take part in gas exchange. Further divisions of the airways lead to the respiratory bronchioles, and eventually to the alveoli. The alveolated region of the lung is where the gas exchange takes place, and is referred to as the respiratory zone. See "Respiratory Physiology", by John B. West, Lippincott Williams & Wilkins, 9th edition published 2011.

A range of respiratory disorders exist. Some examples of respiratory disorders include: Obstructive Sleep Apnea (OSA), Cheyne Stokes Respiration (CSR), Obesity Hyperventilation Syndrome (OHS), Chronic Obstructive Pulmonary Disease (COPD), Neuromuscular Disease (NMD) or chest wall disorders.

Otherwise healthy individuals may take advantage of systems and devices to prevent respiratory disorders from arising.

### 1.2.2 Therapy

Nasal Continuous Positive Airway Pressure (CPAP) therapy has been used to treat Obstructive Sleep Apnea (OSA). The hypothesis is that continuous positive airway pressure acts as a pneumatic splint and may prevent upper airway occlusion by pushing the soft palate and tongue forward and away from the posterior oropharyngeal wall.

Non-invasive ventilation (NIV) provides ventilator support to a patient through the upper airways to assist the patient in taking a full breath and/or maintain adequate oxygen levels in the body by doing some or all of the work of breathing. The ventilator support is provided via a patient interface. NIV has been used to treat CSR, OHS, COPD, MD and Chest Wall disorders.

Invasive ventilation (IV) provides ventilatory support to patients that are no longer able to effectively breathe themselves and is provided using a tracheostomy tube.

Ventilators may control the timing and pressure of breaths pumped into the patient and monitor the breaths taken by the patient. The methods of control and monitoring patients typically include volume-cycled and pressure-cycled methods. The volume-cycled methods may include among others, Pressure-Regulated Volume Control (PRVC), Volume Ventilation (VV), and Volume Controlled Continuous Mandatory Ventilation (VC-CMV) techniques. The pressure-cycled methods may involve, among others, Assist Control (AC), Synchronized Intermittent Mandatory Ventilation (SIMV), Controlled Mechanical Ventilation (CMV), Pressure Support Ventilation (PSV), Continuous Positive Airway Pressure (CPAP), or Positive End Expiratory Pressure (PEEP) techniques.

### 1.2.3 Systems

A treatment system may comprise a Respiratory Pressure Therapy Device (RPT device), an air circuit, a humidifier, a patient interface, and data management.

### 1.2.4 Patient Interface

A patient interface may be used to interface respiratory equipment to its user, for example by providing a flow of breathable gas. The flow of breathable gas may be provided via a mask to the nose and/or mouth, a tube to the mouth or a tracheostomy tube to the trachea of the user. Depending upon the therapy to be applied, the patient interface may form a seal, e.g. with a face region of the patient, to facilitate the delivery of gas at a pressure at sufficient variance with ambient pressure to effect therapy, e.g. a positive pressure of about 10cmH2O. For other forms of therapy, such as the delivery of oxygen, the patient interface may not include a seal sufficient to facilitate delivery to the airways of a supply of gas at a positive pressure of about 10cmH2O.

The design of a patient interface presents a number of challenges. The face has a complex three-dimensional shape. The size and shape of noses varies considerably between individuals. Since the head includes bone, cartilage and soft tissue, different regions of the face respond differently to mechanical forces. The jaw or mandible may move relative to other bones of the skull. The whole head may move during the course of a period of respiratory therapy.

As a consequence of these challenges, some masks suffer from being one or more of obtrusive, aesthetically undesirable, costly, poorly fitting, difficult to use and uncomfortable especially when worn for long periods of time or when a patient is unfamiliar with a system. For example, masks designed solely for aviators, mask designed as part of personal protection equipment (e.g. filter masks), SCUBA masks or for the administration of anaesthetics may be tolerable for their original application, but nevertheless be undesirably uncomfortable to be worn for extended periods of time, e.g. several hours. This is even more so if the mask is to be worn during sleep.

Nasal CPAP therapy is highly effective to treat certain respiratory disorders, provided patients comply with therapy. If a mask is uncomfortable, or difficult to use a patient may not comply with therapy. Since it is often recommended that a patient regularly wash their mask, if a mask is difficult to clean (e.g. difficult to assemble or disassemble), patients may not clean their mask and this may impact on patient compliance.

While a mask for other applications (e.g. aviators) may not be suitable for use in treating sleep disordered breathing, a mask designed for use in treating sleep disordered breathing may be suitable for other applications.

For these reasons, masks for delivery of nasal CPAP during sleep form a distinct field.

### 1.2.4.1 Seal-forming portion

Patient interfaces may include a seal-forming portion. Since it is in direct contact with the patient's face, the shape and configuration of the seal-forming portion can have a direct impact the effectiveness and comfort of the patient interface.

A patient interface may be partly characterised according to the design intent of where the seal-forming portion is to engage with the face in use. In one form of patient interface, a seal-forming portion may comprise two sub-portions to engage with respective left and right nares. In one form of patient interface, a seal-forming portion may comprise a single element that surrounds both nares in use. Such single element may be designed to for example overlay an upper lip region and a nasal bridge region of a face. In one form of patient interface a seal-forming portion may comprise an element that surrounds a mouth region in use, e.g. by forming a seal on a lower lip region of a face. In one form of patient interface, a seal-forming portion may comprise a single element that surrounds both nares and a mouth region in use. These different types of patient interfaces may be known by a variety of names by their manufacturer including nasal masks, full-face masks, nasal pillows, nasal puffs and oro-nasal masks.

A seal-forming portion that may be effective in one region of a patient's face may be in appropriate in another region, e.g. because of the different shape, structure, variability and sensitivity regions of the patient's face. For example, a seal on swimming goggles that overlays a patient's forehead may not be appropriate to use on a patient's nose.

Certain seal-forming portions may be designed for mass manufacture such that one design fit and be comfortable and effective for a wide range of different face shapes and sizes. To the extent to which there is a mismatch between the shape of the patient's face, and the seal-forming portion of the mass-manufactured patient interface, one or both must adapt in order for a seal to form.

One type of seal-forming portion extends around the periphery of the patient interface, and is intended to seal against the user's face when force is applied to the patient interface with the seal-forming portion in confronting engagement with the user's face. The seal-forming portion may include an air or fluid filled cushion, or a moulded or formed surface of a resilient seal element made of an elastomer such as a rubber. With this type of seal-forming portion, if the fit is not adequate, there will be gaps between the seal-forming portion and the face, and additional force will be required to force the patient interface against the face in order to achieve a seal.

Another type of seal-forming portion incorporates a flap seal of thin material so positioned about the periphery of the mask so as to provide a self-sealing action against the face of the user when positive pressure is applied within the mask. Like the previous style of seal forming portion, if the match between the face and the mask is not good, additional force may be required to affect a seal, or the mask may leak. Furthermore, if the shape of the seal-forming portion does not match that of the patient, it may crease or buckle in use, giving rise to leaks.

Another type of seal-forming portion may comprise a friction-fit element, e.g. for insertion into a naris.

Another form of seal-forming portion may use adhesive to affect a seal. Some patients may find it inconvenient to constantly apply and remove an adhesive to their face.

A range of patient interface seal-forming portion technologies are disclosed in the following patent applications, assigned to ResMed Limited: WO 1998/004,310; WO 2006/074,513; WO 2010/135,785.

One form of nasal pillow is found in the Adam Circuit manufactured by Puritan Bennett. Another nasal pillow, or nasal puff is the subject of US Patent 4,782,832 (Trimble et al.), assigned to Puritan-Bennett Corporation.

ResMed Limited has manufactured the following products that incorporate nasal pillows: SWIFT nasal pillows mask, SWIFT II nasal pillows mask, SWIFT LT nasal pillows mask, SWIFT FX nasal pillows mask and LIBERTY full-face mask. The following patent applications, assigned to ResMed Limited, describe nasal pillows masks: International Patent Application WO2004/073,778 (describing amongst other things aspects of ResMed SWIFT nasal pillows), US Patent Application 2009/0044808 (describing amongst other things aspects of ResMed SWIFT LT nasal pillows); International Patent Applications WO 2005/063,328 and WO 2006/130,903 (describing amongst other things aspects of ResMed LIBERTY full-face mask); International Patent Application WO 2009/052,560 (describing amongst other things aspects of ResMed SWIFT FX nasal pillows).

### 1.2.4.2 Positioning and stabilising

A seal-forming portion of a patient interface used for positive air pressure therapy is subject to the corresponding force of the air pressure to disrupt a seal. Thus a variety of techniques have been used to position the seal-forming portion, and to maintain it in sealing relation with the appropriate portion of the face.

One technique is the use of adhesives. See for example US Patent publication US 2010/0000534.

Another technique is the use of one or more straps and stabilising harnesses. Many such harnesses suffer from being one or more of ill-fitting, bulky, uncomfortable and awkward to use.

### 1.2.5 Respiratory Pressure Therapy (RPT) Device

One known RPT device used for treating sleep disordered breathing is the S9 Sleep Therapy System, manufactured by ResMed. Another example of an RPT device is a ventilator. Ventilators such as the ResMed Stellar^{™} Series of Adult and Paediatric Ventilators may provide support for invasive and non-invasive non-dependent ventilation for a range of patients for treating a number of conditions such as but not limited to NMD, OHS and COPD. RPT devices have also been known as flow generators.

The ResMed Elisée^{™} 150 ventilator and ResMed VS III^{™} ventilator may provide support for invasive and non-invasive dependent ventilation suitable for adult or paediatric patients for treating a number of conditions. These ventilators provide volumetric and barometric ventilation modes with a single or double limb circuit.

RPT devices typically comprise a pressure generator, such as a motor-driven blower or a compressed gas reservoir, and are configured to supply a flow of air to the airway of a patient. In some cases, the flow of air may be supplied to the airway of the patient at positive pressure. The outlet of the RPT device is connected via an air circuit to a patient interface such as those described above.

RPT devices typically also include an inlet filter, various sensors and a microprocessor-based controller. A blower may include a servo-controlled motor, a volute and an impeller. In some cases a brake for the motor may be implemented to more rapidly reduce the speed of the blower so as to overcome the inertia of the motor and impeller. The braking can permit the blower to more rapidly achieve a lower pressure condition in time for synchronization with expiration despite the inertia. In some cases the pressure generator may also include a valve capable of discharging generated air to atmosphere as a means for altering the pressure delivered to the patient as an alternative to motor speed control. The sensors measure, amongst other things, motor speed, mass flow rate and outlet pressure, such as with a pressure transducer or the like. The controller may include data storage capacity with or without integrated data retrieval and display functions.

Table of noise output levels of prior devices (one specimen only, measured using test method specified in ISO3744 in CPAP mode at 10cmH₂O).

| Device name | A-weighted sound power level dB(A) | Year (approx.) |
|---|---|---|
| C-Series Tango | 31.9 | 2007 |
| C-Series Tango with Humidifier | 33.1 | 2007 |
| S8 Escape II | 30.5 | 2005 |
| S8 Escape II with H4i Humidifier | 31.1 | 2005 |
| S9 AutoSet | 26.5 | 2010 |
| S9 AutoSet with H5i Humidifier | 28.6 | 2010 |

### 1.2.6 Humidifier

Delivery of a flow of breathable gas without humidification may cause drying of airways. Medical humidifiers are used to increase humidity and/or temperature of the flow of breathable gas in relation to ambient air when required, typically where the patient may be asleep or resting (e.g. at a hospital). As a result, a medical humidifier is preferably small for bedside placement, and it is preferably configured to only humidify and/or heat the flow of breathable gas delivered to the patient without humidifying and/or heating the patient's surroundings. Room-based systems (e.g. a sauna, an air conditioner, an evaporative cooler), for example, may also humidify air that is breathed in by the patient, however they would also humidify and/or heat the entire room, which may cause discomfort to the occupants.

The use of a humidifier with a flow generator or RPT device and the patient interface produces humidified gas that minimizes drying of the nasal mucosa and increases patient airway comfort. In addition, in cooler climates warm air applied generally to the face area in and about the patient interface is more comfortable than cold air.

Respiratory humidifiers are available in many forms and may be a standalone device that is coupled to a respiratory apparatus via an air circuit, is integrated with or configured to be coupled to the relevant respiratory apparatus. While known passive humidifiers can provide some relief, generally a heated humidifier may be used to provide sufficient humidity and temperature to the air so that the patient will be comfortable. Humidifiers typically comprise a water reservoir or tub having a capacity of several hundred milliliters (ml), a heating element for heating the water in the reservoir, a control to enable the level of humidification to be varied, a gas inlet to receive gas from the flow generator or RPT device, and a gas outlet adapted to be connected to an air circuit that delivers the humidified gas to the patient interface.

Heated passover humidification is one common form of humidification used with a RPT device. In such humidifiers the heating element may be incorporated in a heater plate which sits under, and is in thermal contact with, the water tub. Thus, heat is transferred from the heater plate to the water reservoir primarily by conduction. The air flow from the RPT device passes over the heated water in the water tub resulting in water vapour being taken up by the air flow. The ResMed H4i^{™} and H5i^{™} Humidifiers are examples of such heated passover humidifiers that are used in combination with ResMed S8 and S9 CPAP devices respectively.

Other humidifiers may also be used such as a bubble or diffuser humidifier, a jet humidifier or a wicking humidifier. In a bubble or diffuser humidifier the air is conducted below the surface of the water and allowed to bubble back to the top. A jet humidifier produces an aerosol of water and baffles or filters may be used so that the particles are either removed or evaporated before leaving the humidifier. A wicking humidifier uses a water absorbing material, such as sponge or paper, to absorb water by capillary action. The water absorbing material is placed within or adjacent at least a portion of the air flow path to allow evaporation of the water in the absorbing material to be taken up into the air flow.

An alternative form of humidification is provided by the ResMed HumiCare^{™} D900 humidifier that uses a CounterStream^{™} technology that directs the air flow over a large surface area in a first direction whilst supplying heated water to the large surface area in a second opposite direction. The ResMed HumiCare^{™} D900 humidifier may be used with a range of invasive and non-invasive ventilators.

Systems and methods for transmitting patient usage data are known from US 2014/032231 A1, US 2003/236450 A1, WO 2013/002650 A1 and US 2013/269700 A1.

### 2 BRIEF SUMMARY OF THE TECHNOLOGY

According to the invention, there is provided a system according to claim 1 and a method according to claim 14. The dependent claims define embodiments of the invention as claimed.

Aspects of the disclosure provide a computer implemented method for management of patient data. The method may include accessing a subscription that may include a set of instructions and collecting usage data for a patient device, wherein the usage data relates to a patient's use of the patient device. The method may also include determining that a triggering event has occurred and transmitting at least a portion of the collected usage data in accordance with the subscription. At least one of the following; collecting the usage data, determining the triggering event and transmitting at least a portion of the collected usage data, is performed in accordance with the accessed subscription.

The subscription may identify a plurality of conditions to be met before the usage is to be transmitted, and the triggering event may include a determination that each of the conditions has been met. The step of transmitting at least a portion of the collected usage data may include transmitting the set of usage data. The patient device may include a respiratory pressure therapy device.

In one example, the triggering event may be based on a patient having finished using the patient device for a predetermined period of time. In addition, the usage data may identify time periods in which the patient device has been used. The usage data may also relate to at least one of a patient's apnea index, hypopnea index, and apnea-hypopnea index.

In another aspect, a method for managing patient data is disclosed that includes receiving transmissions of usage data from a plurality of patient devices, wherein each transmission of usage data has occurred in accordance with a triggering event identified in a set of instructions. The method also includes storing the usage data in a memory, wherein the usage data is stored so as to be associated with each patient device, of the plurality of patient devices, from which the transmission was received. The method may also include receiving, a request for at least a portion of the stored usage data, and transmitting the requested portion of the stored usage data.

The set of instructions may identify a plurality of conditions to be met before the usage is to be transmitted, and the triggering event may include a determination that each of the conditions has been met. In one example, receiving transmissions of usage data may include receiving a first set of usage data from a first patient device and receiving a second set of usage data from a second device, wherein the first set of usage data may be transmitted in accordance with a first triggering event and the second set of usage data may be transmitted in accordance with a second triggering event. The first triggering event and the second triggering event may be based on different criteria.

In one example, the request for at least a portion of the stored usage data identifies a first patient, from the plurality of patients, and the portion of the stored usage data may be associated with the first patient. In addition, the set of instructions may identify the triggering event as a patient having finished using the patient device for a predetermined period of time. The usage data may also relate to the period of time for which each patient device, of the plurality of patient devices, has been used. In some aspects the term "usage data" may be understood to include any one of the following; therapeutic data, prescription and comfort settings, faults, logs, humidity data, temperature data, or any other data associated with the configuration, operation, the use and the ambient environment of the device.

In another aspect, the subscription may be generated based on a user's selection of individual data items that are to be collected by the patient device. The subscription may also identify subsets of usage data and identify one or more triggering events for each subset of usage data. In addition, the usage data to be collected in accordance with the subscription may change over time. The triggering events may include a change in one or more settings of the patient device or a fault condition in one or more components of a patient device.

The disclosure also provides for a system for managing patient data, wherein the system includes a one or more computing devices configured to perform the methods described herein.

### 3 BRIEF DESCRIPTION OF THE SEVERAL VIEWS OF THE DRAWINGS

The present technology is illustrated by way of example, and not by way of limitation, in the figures of the accompanying drawings, in which like reference numerals refer to similar elements including:

### 3.1 TREATMENT SYSTEMS

Fig. 1a shows a system in accordance with the present technology. A patient 1000 wearing a patient interface 3000, in the form of nasal pillows, receives a supply of air at positive pressure from a RPT device 4000. Air from the RPT device is humidified in a humidifier 5000, and passes along an air circuit 4170 to the patient 1000.
Fig. 1b shows a system including a patient 1000 wearing a patient interface 3000, in the form of a nasal mask, receives a supply of air at positive pressure from a RPT device 4000. Air from the RPT device is humidified in a humidifier 5000, and passes along an air circuit 4170 to the patient 1000.
Fig. 1c shows a system including a patient 1000 wearing a patient interface 3000, in the form of a full-face mask, receives a supply of air at positive pressure from a RPT device. Air from the RPT device is humidified in a humidifier 5000, and passes along an air circuit 4170 to the patient 1000.

### 3.2 THERAPY

### 3.2.1 Respiratory system

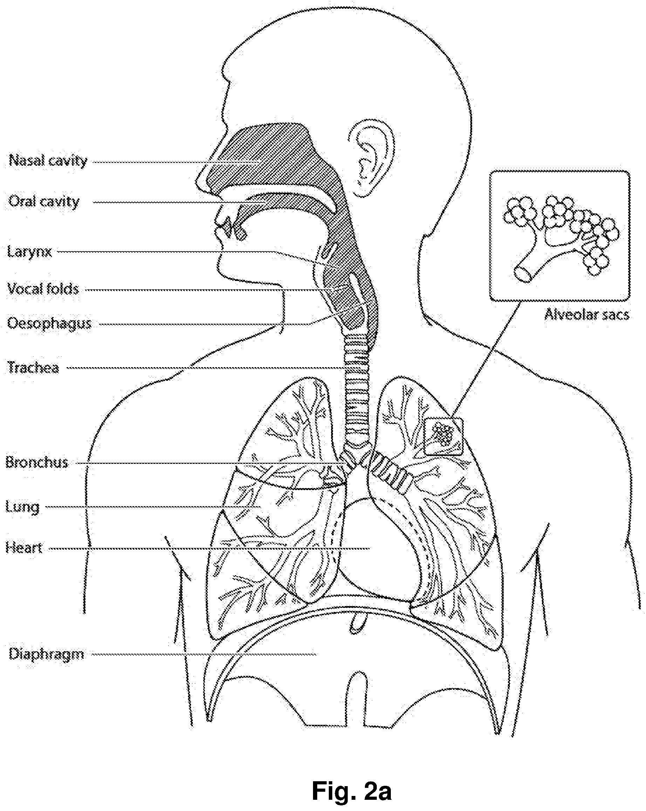

Fig. 2a shows an overview of a human respiratory system including the nasal and oral cavities, the larynx, vocal folds, oesophagus, trachea, bronchus, lung, alveolar sacs, heart and diaphragm.

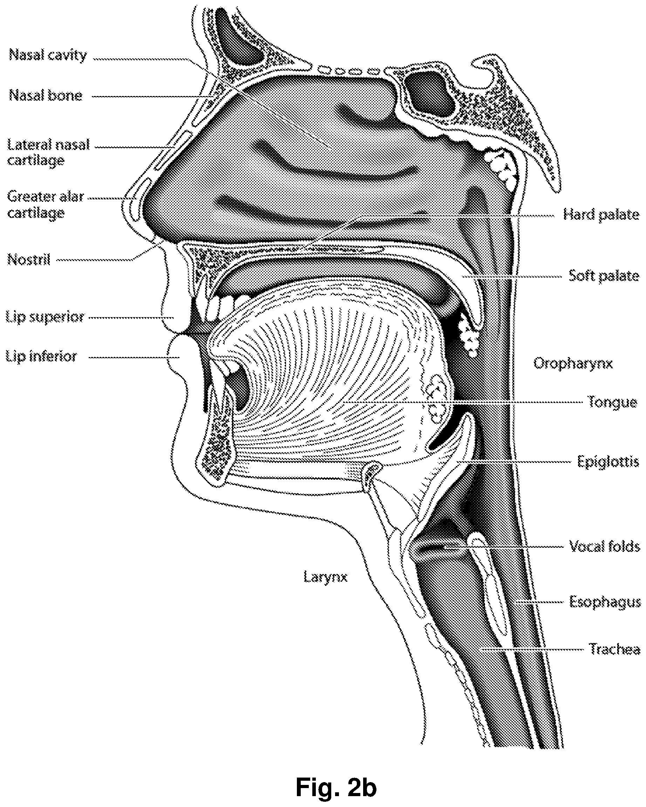

Fig. 2b shows a view of a human upper airway including the nasal cavity, nasal bone, lateral nasal cartilage, greater alar cartilage, nostril, lip superior, lip inferior, larynx, hard palate, soft palate, oropharynx, tongue, epiglottis, vocal folds, oesophagus and trachea.

### 3.2.2 Facial anatomy

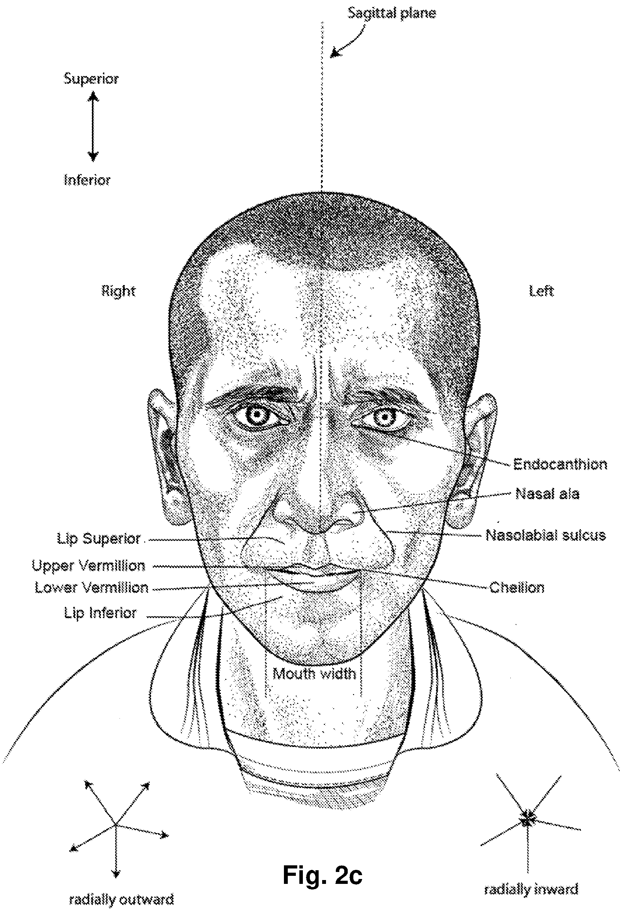

Fig. 2c is a front view of a face with several features of surface anatomy identified including the lip superior, upper vermillion, lower vermillion, lip inferior, mouth width, endocanthion, a nasal ala, nasolabial sulcus and cheilion.

### 3.3 PATIENT INTERFACE

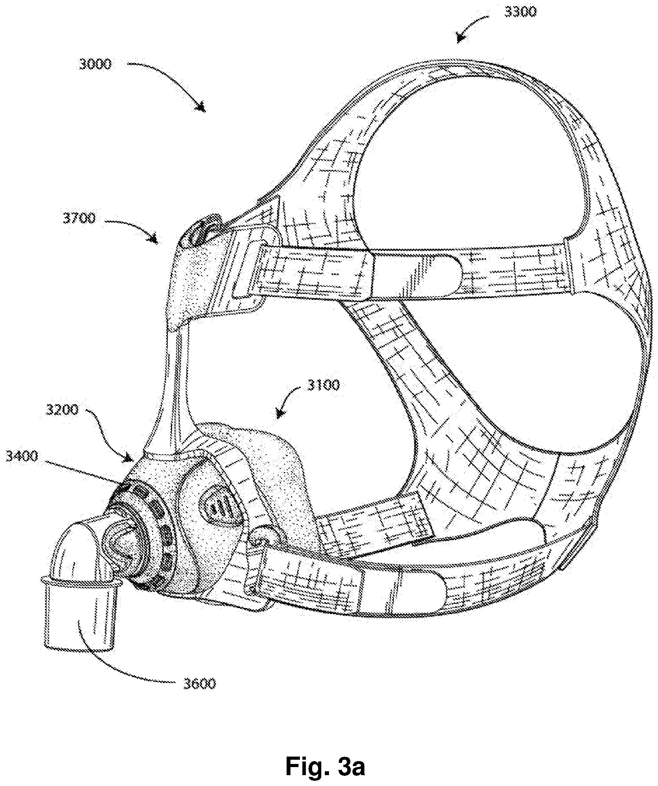

Fig. 3a shows an example of a patient interface known in the prior art.

### 3.4 RESPIRATORY PRESSURE THERAPY (RPT) DEVICE

Fig. 4a shows a RPT device in accordance with one form of the present technology.
Fig. 4b shows a schematic diagram of the pneumatic circuit of a RPT device in accordance with one form of the present technology. The directions of upstream and downstream are indicated.
Fig. 4c shows a schematic diagram of the electrical components of a RPT device in accordance with one aspect of the present technology.

### 3.5 HUMIDIFIER

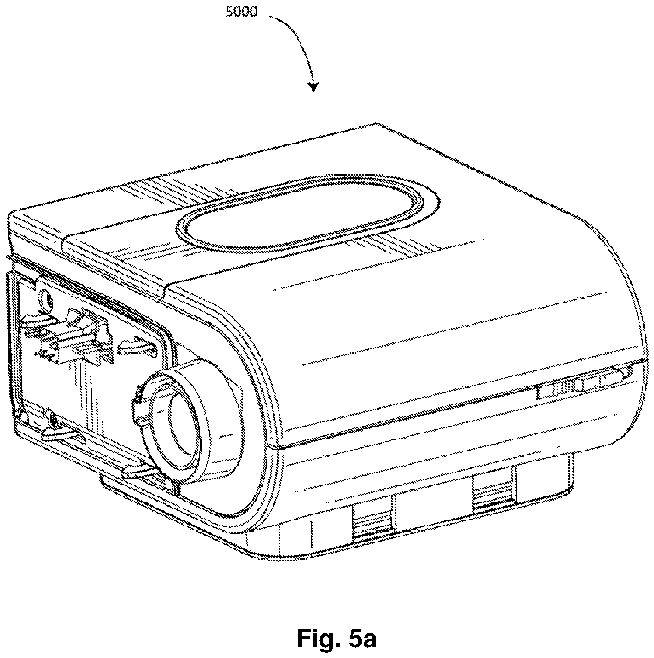

Fig. 5a shows a humidifier in accordance with one aspect of the present technology.

### 3.6 BREATHING WAVEFORMS

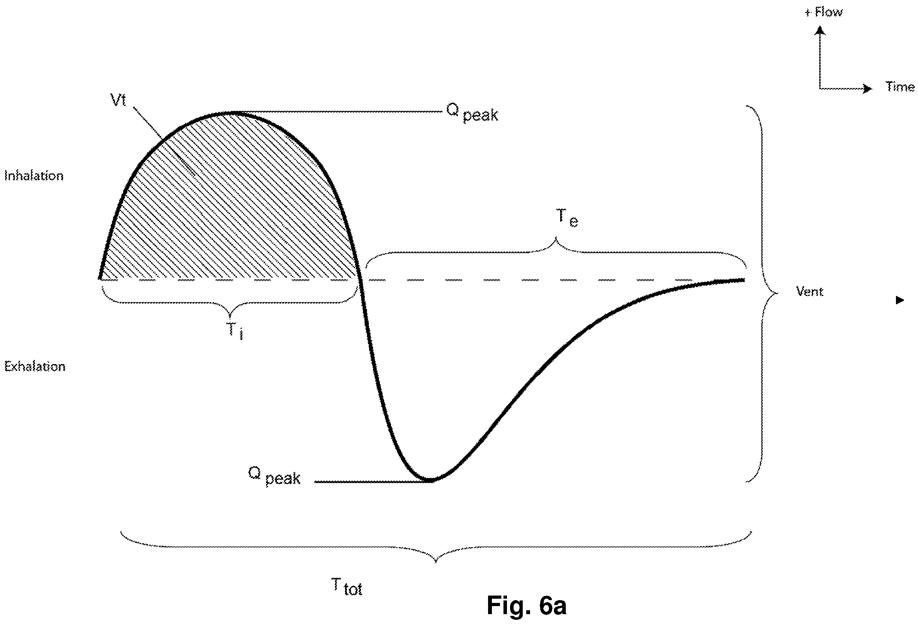

Fig. 6a shows a model typical breath waveform of a person while sleeping, the horizontal axis is time, and the vertical axis is respiratory flow. While the parameter values may vary, a typical breath may have the following approximate values: tidal volume, Vt, 0.5L, inhalation time, Ti, 1.6s, peak inspiratory flow, Qpeak, 0.4 L/s, exhalation time, Te, 2.4s, peak expiratory flow, Qpeak, -0.5 L/s. The total duration of the breath, Ttot, is about 4s. The person typically breathes at a rate of about 15 breaths per minute (BPM), with Ventilation, Vent, about 7.5 L/s. A typical duty cycle, the ratio of Ti to Ttot is about 40%.

### 3.7 DATA MANAGEMENT SYSTEM

Fig. 7 shows an example communications system 700 that may be used in the collection and transmission of patient data. Each patient device 720, 730, and 740 may comprise an RPT 4000, humidifier 5000, patient interface 3000. Fig. 8 shows a flow diagram 800 of operations that may be performed by patient devices disclosed herein in connection with the collection and transmission of patient data.

### 4 DETAILED DESCRIPTION OF EXAMPLES OF THE TECHNOLOGY

Before the present technology is described in further detail, it is to be understood that the technology is not limited to the particular examples described herein, which may vary. It is also to be understood that the terminology used in this disclosure is for the purpose of describing only the particular examples discussed herein, and is not intended to be limiting.

### 4.1 TREATMENT SYSTEMS

In one form, the present technology comprises apparatus for treating a respiratory disorder. The apparatus may comprise a flow generator or blower for supplying pressurised respiratory gas, such as air, to the patient 1000 via an air delivery tube leading to a patient interface 3000.

### 4.2 THERAPY

In one form, the present technology comprises a method for treating a respiratory disorder comprising the step of applying positive pressure to the entrance of the airways of a patient 1000.

### 4.2.1 Nasal CPAP for OSA

In one form, the present technology comprises a method of treating Obstructive Sleep Apnea in a patient by applying nasal continuous positive airway pressure to the patient.

### 4.3 PATIENT INTERFACE 3000

A non-invasive patient interface 3000 in accordance with one aspect of the present technology comprises the following functional aspects: a seal-forming structure 3100, a plenum chamber 3200, a positioning and stabilising structure 3300, a vent 3400 and a connection port 3600 for connection to air circuit 4170. In some forms a functional aspect may be provided by one or more physical components. In some forms, one physical component may provide one or more functional aspects. In use the seal-forming structure 3100 is arranged to surround an entrance to the airways of the patient so as to facilitate the supply of air at positive pressure to the airways.

### 4.3.1 Seal-forming structure 3100

In one form of the present technology, a seal-forming structure 3100 provides a sealing-forming surface, and may additionally provide a cushioning function.

A seal-forming structure 3100 in accordance with the present technology may be constructed from a soft, flexible, resilient material such as silicone.

In one form, the seal-forming structure 3100 comprises a sealing flange and a support flange. Preferably the sealing flange comprises a relatively thin member with a thickness of less than about 1mm, for example about 0.25mm to about 0.45mm, that extends around the perimeter 3210 of the plenum chamber 3200. Support flange may be relatively thicker than the sealing flange. The support flange is disposed between the sealing flange and the marginal edge of the plenum chamber 3200, and extends at least part of the way around the perimeter 3210. The support flange is or includes a spring-like element and functions to support the sealing flange from buckling in use. In use the sealing flange can readily respond to system pressure in the plenum chamber 3200 acting on its underside to urge it into tight sealing engagement with the face.

In one form the seal-forming portion of the non-invasive patient interface 3000 comprises a pair of nasal puffs, or nasal pillows, each nasal puff or nasal pillow being constructed and arranged to form a seal with a respective naris of the nose of a patient.

Nasal pillows in accordance with an aspect of the present technology include: a frusto-cone, at least a portion of which forms a seal on an underside of the patient's nose; a stalk, a flexible region on the underside of the cone and connecting the cone to the stalk. In addition, the structure to which the nasal pillow of the present technology is connected includes a flexible region adjacent the base of the stalk. The flexible regions can act in concert to facilitate a universal joint structure that is accommodating of relative movement- both displacement and angular- of the frusto-cone and the structure to which the nasal pillow is connected. For example, the frusto-cone may be axially displaced towards the structure to which the stalk is connected.

In one form the non-invasive patient interface 3000 comprises a seal-forming portion that forms a seal in use on an upper lip region (that is, the lip superior) of the patient's face.

In one form the non-invasive patient interface 3000 comprises a seal-forming portion that forms a seal in use on a chin-region of the patient's face.

### 4.3.2 Plenum chamber 3200

Preferably the plenum chamber 3200 has a perimeter 3210 that is shaped to be complementary to the surface contour of the face of an average person in the region where a seal will form in use. In use, a marginal edge of the plenum chamber 3200 is positioned in close proximity to an adjacent surface of the face. Actual contact with the face is provided by the seal-forming structure 3100. Preferably the seal-forming structure 3100 extends in use about the entire perimeter 3210 of the plenum chamber 3200.

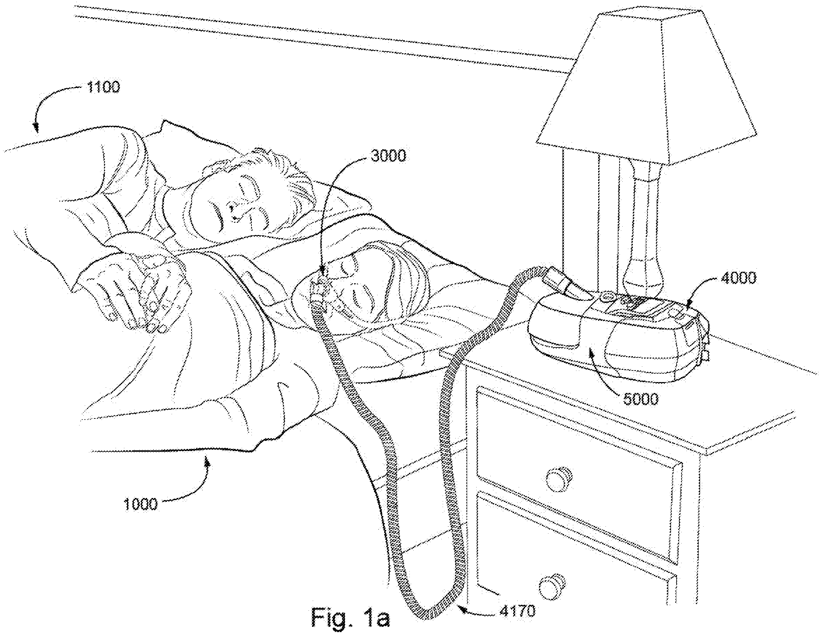

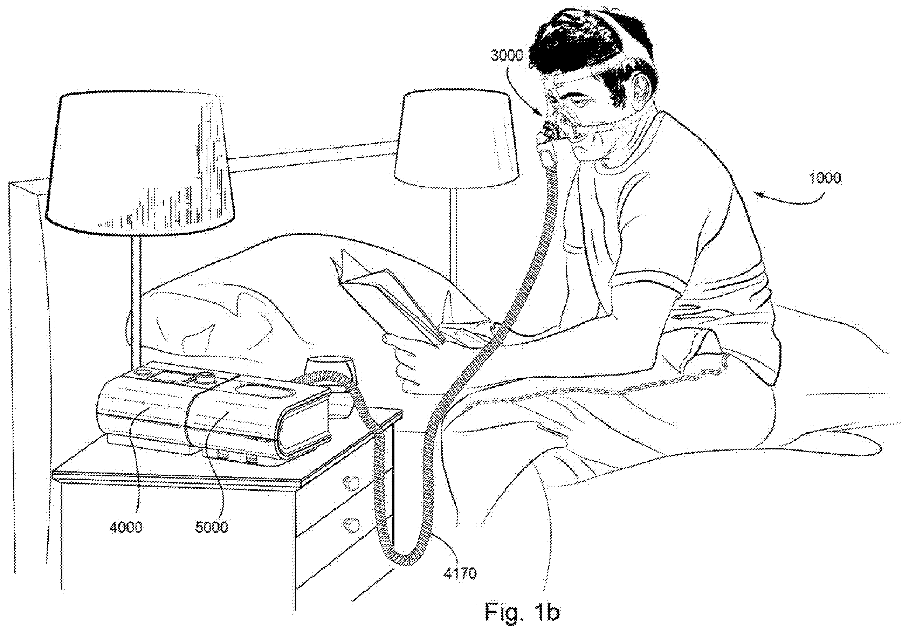

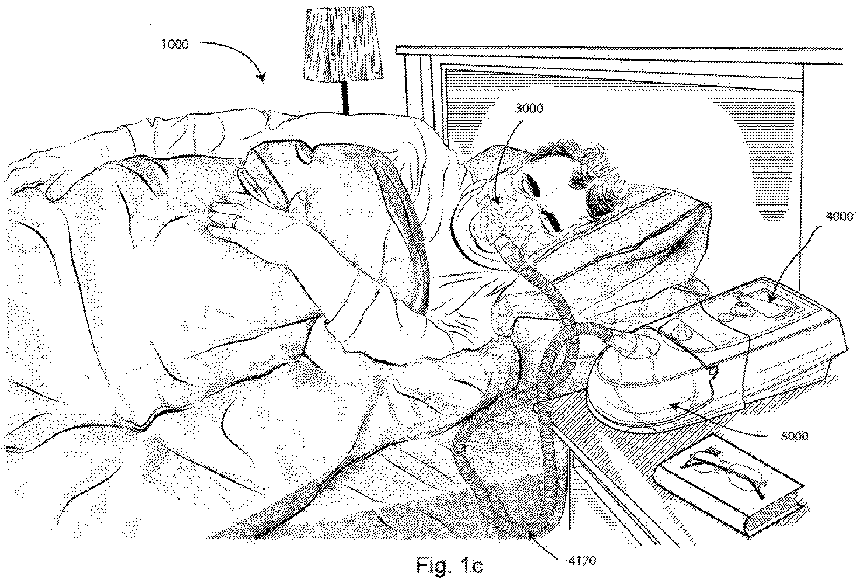

In one form, the plenum chamber 3200 may surround and/or be in fluid communication with the nares of the patient where the plenum chamber 3200 is a part of a nasal mask (e.g. shown in Fig 1b). In another form, the plenum chamber 3200 may surround and/or be in fluid communication with the nares and the mouth of the patient where the plenum chamber 3200 is a part of a full-face mask (e.g., shown in Fig. 1c). In yet another form, the plenum chamber 3200 may engage and/or be in fluid communication with one or more of the nares of the patient where the plenum chamber 3200 is a part of nasal pillows (e.g., shown in Fig. 29).

### 4.3.3 Positioning and stabilising structure 3300

Preferably the seal-forming structure 3100 of the patient interface 3000 of the present technology is held in sealing position in use by the positioning and stabilising structure 3300.

### 4.4 RPT DEVICE 4000

An example RPT device 4000 that may be suitable for implementing aspects of the present technology may include mechanical and pneumatic components 4100, electrical components 4200 and may be programmed to execute one or more of the control methodologies or algorithms described throughout this specification. The RPT device may have an external housing 4010, preferably formed in two parts, an upper portion 4012 of the external housing 4010, and a lower portion 4014 of the external housing 4010. In alternative forms, the external housing 4010 may include one or more panel(s) 4015. Preferably the RPT device 4000 comprises a chassis 4016 that supports one or more internal components of the RPT device 4000. In one form a pneumatic block 4020 is supported by, or formed as part of the chassis 4016. The RPT device 4000 may include a handle 4018.

The pneumatic path of the RPT device 4000 preferably comprises an inlet air filter 4112, an inlet muffler 4122, a controllable pressure device 4140 capable of supplying air at positive pressure (preferably a blower 4142), and an outlet muffler 4124. One or more pressure sensors 4271 and flow sensors 4274 are included in the pneumatic path.

The preferred pneumatic block 4020 comprises a portion of the pneumatic path that is located within the external housing 4010.

The RPT device 4000 preferably has an electrical power supply 4210, one or more input devices 4220, a central controller 4230, a therapy device controller 4240 and/or any of the controllers previously described, a pressure device 4140, one or more protection circuits 4250, memory 4260, transducers 4270, data communication interface 4280 and one or more output devices 4290. Electrical components 4200 may be mounted on a single Printed Circuit Board Assembly (PCBA) 4202. In an alternative form, the RPT device 4000 may include more than one PCBA 4202.

The central controller 4230 of the RPT device 4000, which may include one or more processors, can be programmed to execute one or more algorithm modules, preferably including a pre-processing module, a therapy engine module, a pressure control module, and further preferably a fault condition module. It may further include a vent control module that may be configured with one or more of the vent control methodologies described throughout this specification.

### 4.4.1 RPT device mechanical & pneumatic components 4100

### 4.4.1.1 Air filter(s) 4110

A RPT device in accordance with one form of the present technology may include an air filter 4110, or a plurality of air filters 4110.

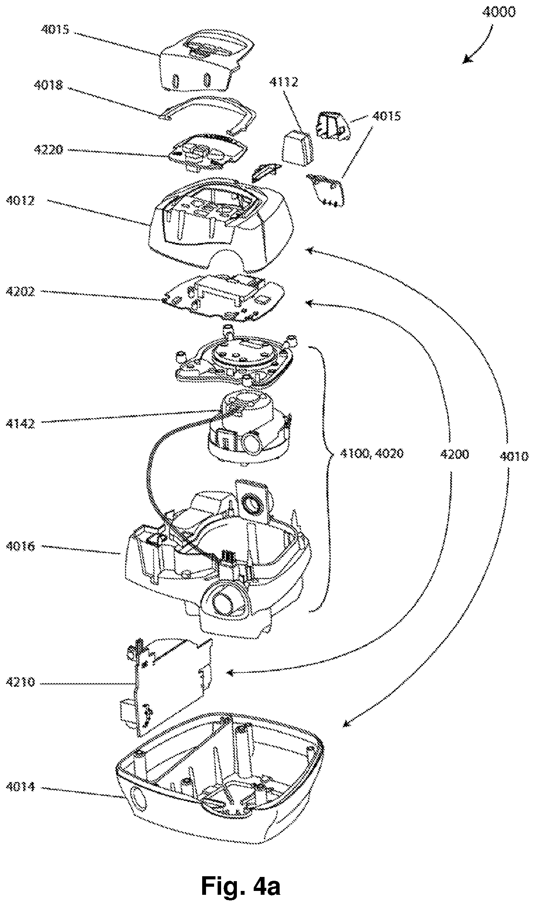

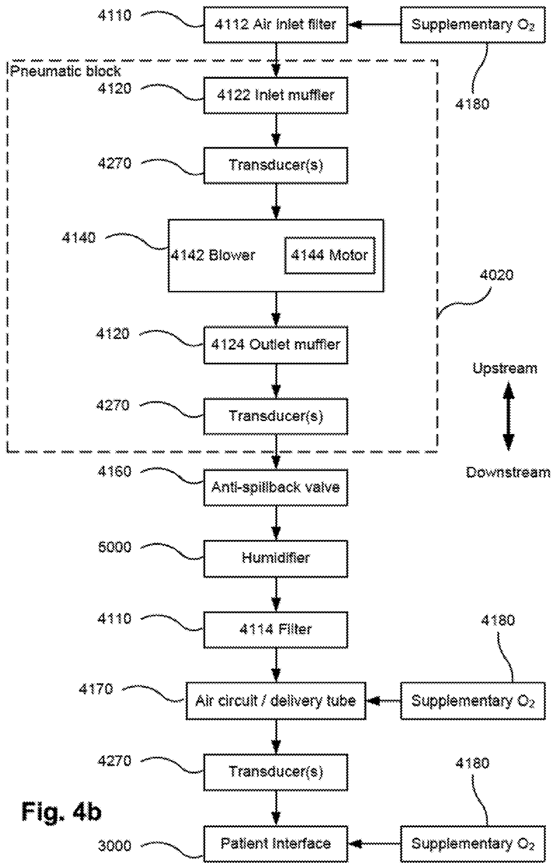

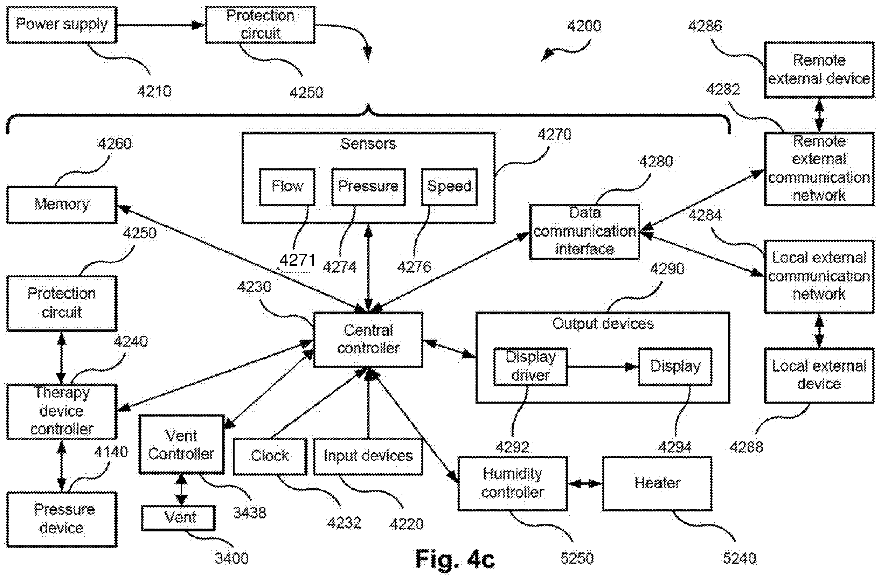

In one form, an inlet air filter 4112 is located at the beginning of the pneumatic path upstream of a blower 4142. See Fig. 4b.

In one form, an outlet air filter 4114, for example an antibacterial filter, is located between an outlet of the pneumatic block 4020 and a patient interface 3000. See Fig. 4b.

### 4.4.1.2 Muffler(s) 4120

In one form of the present technology, an inlet muffler 4122 is located in the pneumatic path upstream of a blower 4142. See Fig. 4b.

In one form of the present technology, an outlet muffler 4124 is located in the pneumatic path between the blower 4142 and a patient interface 3000. See Fig. 4b.

### 4.4.1.3 Pressure device 4140

In a preferred form of the present technology, a pressure device 4140 for producing a flow of air at positive pressure is a controllable blower 4142. For example the blower may include a brushless DC motor 4144 with one or more impellers housed in a volute. The blower may be preferably capable of delivering a supply of air, for example about 120 litres/minute, at a positive pressure in a range from about 4 cmH₂O to about 20 cmH₂O, or in other forms up to about 30 cmH₂O.

The pressure device 4140 is under the control of the therapy device controller 4240.

### 4.4.1.4 Transducer(s) 4270

In one form of the present technology, one or more transducers 4270 are located upstream of the pressure device 4140. The one or more transducers 4270 are constructed and arranged to measure properties of the air at that point in the pneumatic path.

In one form of the present technology, one or more transducers 4270 are located downstream of the pressure device 4140, and upstream of the air circuit 4170. The one or more transducers 4270 are constructed and arranged to measure properties of the air at that point in the pneumatic path.

In one form of the present technology, one or more transducers 4270 are located proximate to the patient interface 3000.

### 4.4.1.5 Anti-spill back valve 4160

In one form of the present technology, an anti-spill back valve is located between the humidifier 5000 and the pneumatic block 4020. The anti-spill back valve is constructed and arranged to reduce the risk that water will flow upstream from the humidifier 5000, for example to the motor 4144.

### 4.4.1.6 Air circuit 4170

An air circuit 4170 in accordance with an aspect of the present technology is constructed and arranged to allow a flow of air or breathable gasses between the pneumatic block 4020 and the patient interface 3000.

### 4.4.1.7 Oxygen delivery

In one form of the present technology, supplemental oxygen 4180 is delivered to a point in the pneumatic path.

In one form of the present technology, supplemental oxygen 4180 is delivered upstream of the pneumatic block 4020.

In one form of the present technology, supplemental oxygen 4180 is delivered to the air circuit 4170.

In one form of the present technology, supplemental oxygen 4180 is delivered to the patient interface 3000.

### 4.4.2 RPT device electrical components 4200

### 4.4.2.1 Power supply 4210

In one form of the present technology power supply 4210 is internal of the external housing 4010 of the RPT device 4000. In another form of the present technology, power supply 4210 is external of the external housing 4010 of the RPT device 4000.

In one form of the present technology power supply 4210 provides electrical power to the RPT device 4000 only. In another form of the present technology, power supply 4210 provides electrical power to both RPT device 4000 and humidifier 5000. The power supply may also optionally provide power to any actuator, controller and/or sensors for a vent arrangement as described throughout this specification

### 4.4.2.2 Input devices 4220

In one form of the present technology, a RPT device 4000 includes one or more input devices 4220 in the form of buttons, switches or dials to allow a person to interact with the device. These may be implemented for entering settings for operation of the components of the RPT device such as the vent arrangement. The buttons, switches or dials may be physical devices, or software devices accessible via a touch screen. The buttons, switches or dials may, in one form, be physically connected to the external housing 4010, or may, in another form, be in wireless communication with a receiver that is in electrical connection to the central controller 4230.

In one form the input device 4220 may be constructed and arranged to allow a person to select a value and/or a menu option.

### 4.4.2.3 Central controller 4230

In one form of the present technology, the central controller 4230 is a dedicated electronic circuit configured to receive input signal(s) from the input device 4220, and to provide output signal(s) to the output device 4290 and / or the therapy device controller 4240.

In one form, the central controller 4230 is an application-specific integrated circuit. In another form, the central controller 4230 comprises discrete electronic components.

In another form of the present technology, the central controller 4230 is a processor suitable to control a RPT device 4000 such as an x86 INTEL processor.

A processor of a central controller 4230 suitable to control a RPT device 4000 in accordance with another form of the present technology includes a processor based on ARM Cortex-M processor from ARM Holdings. For example, an STM32 series microcontroller from ST MICROELECTRONICS may be used.

Another processor suitable to control a RPT device 4000 in accordance with a further alternative form of the present technology includes a member selected from the family ARM9-based 32-bit RISC CPUs. For example, an STR9 series microcontroller from ST MICROELECTRONICS may be used.

In certain alternative forms of the present technology, a 16-bit RISC CPU may be used as the processor for the RPT device 4000. For example a processor from the MSP430 family of microcontrollers, manufactured by TEXAS INSTRUMENTS, may be used.

The processor is configured to receive input signal(s) from one or more transducers 4270, and one or more input devices 4220.

The processor is configured to provide output signal(s) to one or more of an output device 4290, a therapy device controller 4240, a data communication interface 4280 and humidifier controller 5250.

In some forms of the present technology, the processor of the central controller 4230, or multiple such processors, is configured to implement the one or more methodologies described herein such as the one or more algorithms 4300 expressed as computer programs stored in a non-transitory computer readable storage medium, such as memory 4260. In some cases, as previously discussed, such processor(s) may be integrated with a RPT device 4000. However, in some forms of the present technology the processor(s) may be implemented discretely from the flow generation components of the RPT device 4000, such as for purpose of performing any of the methodologies described herein without directly controlling delivery of a respiratory treatment. For example, such a processor may perform any of the methodologies described herein for purposes of determining control settings for a ventilator or other respiratory related events by analysis of stored data such as from any of the sensors described herein. Similarly, such a processor may perform any of the methodologies described herein for purposes controlling operation of any vent arrangement described in this specification.

### 4.4.2.4 Clock 4232

Preferably RPT device 4000 includes a clock 4232 that is connected to processor.

### 4.4.2.5 Therapy device controller 4240

In one form of the present technology, therapy device controller 4240 is a pressure control module 4330 that forms part of the algorithms 4300 executed by the processor of the central controller 4230.

In one form of the present technology, therapy device controller 4240 is a dedicated motor control integrated circuit. For example, in one form a MC33035 brushless DC motor controller, manufactured by ONSEMI is used.

### 4.4.2.6 Protection circuits 4250

Preferably a RPT device 4000 in accordance with the present technology comprises one or more protection circuits 4250.

One form of protection circuit 4250 in accordance with the present technology is an electrical protection circuit.

One form of protection circuit 4250 in accordance with the present technology is a temperature or pressure safety circuit.

### 4.4.2.7 Memory 4260

In accordance with one form of the present technology the RPT device 4000 includes memory 4260, preferably non-volatile memory. In some forms, memory 4260 may include battery powered static RAM. In some forms, memory 4260 may include volatile RAM.

Preferably memory 4260 is located on PCBA 4202. Memory 4260 may be in the form of EEPROM, or NAND flash.

Additionally or alternatively, RPT device 4000 includes removable form of memory 4260, for example a memory card made in accordance with the Secure Digital (SD) standard.

In one form of the present technology, the memory 4260 acts as a non-transitory computer readable storage medium on which is stored computer program instructions expressing the one or more methodologies described herein, such as the one or more algorithms 4300.

### 4.4.2.8 Transducers 4270

Transducers may be internal of the device, or external of the RPT device. External transducers may be located for example on or form part of the air delivery circuit, e.g. the patient interface. External transducers may be in the form of non-contact sensors such as a Doppler radar movement sensor that transmit or transfer data to the RPT device.

### 4.4.2.8.1 Flow

A flow transducer 4274 in accordance with the present technology may be based on a differential pressure transducer, for example, an SDP600 Series differential pressure transducer from SENSIRION. The differential pressure transducer is in fluid communication with the pneumatic circuit, with one of each of the pressure transducers connected to respective first and second points in a flow restricting element.

In use, a signal representing total flow Qt from the flow transducer 4274 is received by the processor.

### 4.4.2.8.2 Pressure

A pressure transducer 4272 in accordance with the present technology is located in fluid communication with the pneumatic circuit. An example of a suitable pressure transducer is a sensor from the HONEYWELL ASDX series. An alternative suitable pressure transducer is a sensor from the NPA Series from GENERAL ELECTRIC.

In use, a signal from the pressure transducer 4272 is received by the central controller processor. In one form, the signal from the pressure transducer 4272 is filtered prior to being received by the central controller 4230.

### 4.4.2.8.3 Motor speed

In one form of the present technology a motor speed signal 4276 is generated. A motor speed signal 4276 is preferably provided by therapy device controller 4240. Motor speed may, for example, be generated by a speed sensor, such as a Hall effect sensor.

### 4.4.2.9 Data communication interface 4280

In one preferred form of the present technology, a data communication interface 4280 is provided, and is connected to central controller processor. Data communication interface 4280 is preferably connectable to remote external communication network 4282. Data communication interface 4280 is preferably connectable to local external communication network 4284. Preferably remote external communication network 4282 is connectable to remote external device 4286. Preferably local external communication network 4284 is connectable to local external device 4288.

In one form, data communication interface 4280 is part of processor of central controller 4230. In another form, data communication interface 4280 is an integrated circuit that is separate from the central controller processor.

In one form, remote external communication network 4282 is the Internet. The data communication interface 4280 may use wired communication (e.g. via Ethernet, or optical fibre) or a wireless protocol to connect to the Internet.

In one form, local external communication network 4284 utilises one or more communication standards, such as Bluetooth, or a consumer infrared protocol.

In one form, remote external device 4286 is one or more computers, for example a cluster of networked computers. In one form, remote external device 4286 may be virtual computers, rather than physical computers. In either case, such remote external device 4286 may be accessible to an appropriately authorised person such as a clinician.

Preferably local external device 4288 is a personal computer, mobile phone, tablet or remote control.

### 4.4.2.10 Output devices including optional display, alarms

An output device 4290 in accordance with the present technology may take the form of one or more of a visual, audio and haptic unit. A visual display may be a Liquid Crystal Display (LCD) or Light Emitting Diode (LED) display.

### 4.4.2.10.1 Display driver 4292

A display driver 4292 receives as an input the characters, symbols, or images intended for display on the display 4294, and converts them to commands that cause the display 4294 to display those characters, symbols, or images.

### 4.4.2.10.2 Display 4294

A display 4294 is configured to visually display characters, symbols, or images in response to commands received from the display driver 4292. For example, the display 4294 may be an eight-segment display, in which case the display driver 4292 converts each character or symbol, such as the figure "0", to eight logical signals indicating whether the eight respective segments are to be activated to display a particular character or symbol.

### 4.5 COMMUNICATION AND DATA MANAGEMENT SYSTEM

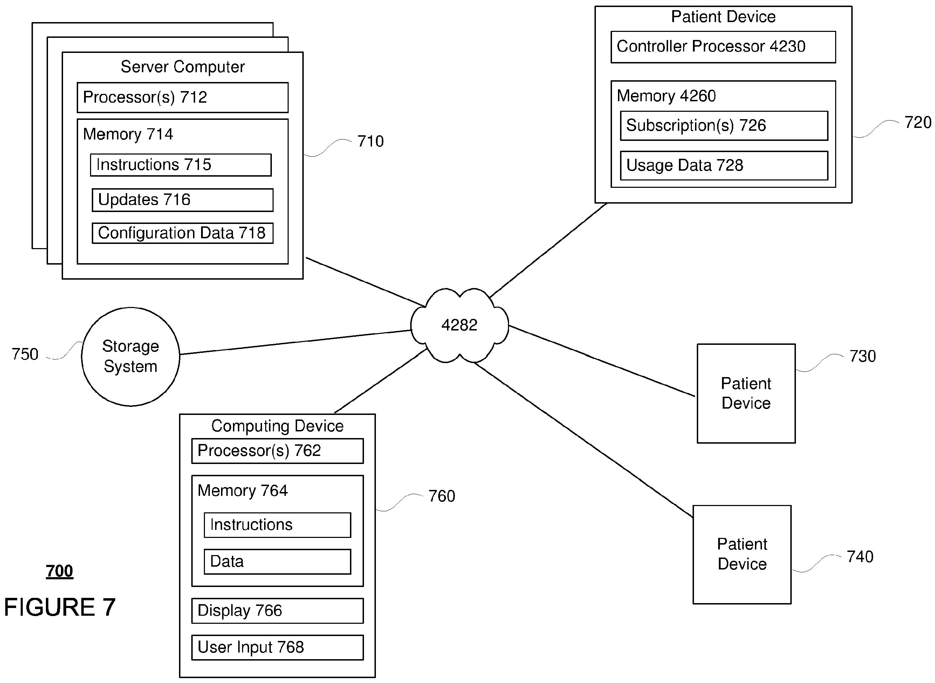

Fig. 7 depicts an example system 700 in which aspects of the disclosure may be implemented. This example should not be considered as limiting the scope of the disclosure or usefulness of the features described herein. In this example, system 700 includes server 710, patient devices 720, 730, and 740, storage systems 750, as well as computing device 760. These devices may each communicate over network 4282.

Each patient device 720, 730, and 740 may include one or more devices, including RPT 4000, humidifier 5000, and patient interface 3000. In addition, each patient device 720, 730, and 740 may be operated at remote locations and by different patients. While only central controller 4230 and memory 4260 are shown in patient device 720, each patient device may include any of the components discussed above in connection with RPT 4000, humidifier 5000, and patient interface 3000. In addition, while patient devices 720, 730, and 740 are shown as communicating directly over 4282, each patient device may also communicate over network 4282 via an external computing device. For example, patient device 720 may communicate with a personal computer that transmits data over network 4282.

Servers 710 contain one or more processors 712, memory 714 and may be incorporated with other components typically present in general purpose computing devices. Memory 714 of server 710 may store information accessible by processor 712, including instructions 715 that can be executed by the processor 712. Memory 714 may also include data 718 that can be retrieved, manipulated or stored by processor 712. The memory can be of any non-transitory type capable of storing information accessible by the processor. The subscriptions 716 may include instructions that are directly or indirectly executed by processor 712. In that regard, the terms "instructions," "application," "steps" and "programs" can be used interchangeably herein. Functions, methods and routines of the instructions are explained in more detail below.

Data 718 may be retrieved, stored or modified by processor 712 in accordance with the instructions 715. For instance, although the subject matter described herein is not limited by any particular data structure, the data can be stored in computer registers, in a relational database as a table having many different fields and records, or XML documents. Data 718 may also be any information sufficient to identify or calculate relevant information, such as numbers, descriptive text, proprietary codes, pointers, references to data stored in other memories such as at other network locations. The one or more processors 712 may include conventional processors, such as a CPU, or may be a hardware-based component, such as an ASIC.

Although Fig. 7 functionally illustrates the processor, memory, and other elements of server 710, computing device 760 and patient devices 720, 730, and 740 as each being within one block, the various components of each device may be stored within different physical housings. For example, memory 714 may be a hard drive or other storage media located in a housing different from that of server 710. Similarly, processor 712 may include a plurality of processors, some or all of which are located in a housing different from that of server 710. Accordingly, references to a processor, computer, computing device, or memory will be understood to include references to a collection of processors, computers, computing devices, or memories that may or may not operate in parallel. Although some functions are described herein as taking place on a single computing device having a single processor, various aspects of the disclosure may be implemented by a plurality of computing devices communicating information with one another, such as by communicating over network 4282.

Network 4282 and intervening nodes described herein can be interconnected using various protocols and systems, such that the network can be part of the Internet, World Wide Web, specific intranets, wide area networks, local networks, or cell phone networks. The network can utilize standard communications protocols, such as Ethernet, Wi-Fi and HTTP, protocols that are proprietary to one or more companies, and various combinations of the foregoing. Although certain advantages are obtained when information is transmitted or received as noted above, other aspects of the subject matter described herein are not limited to any particular manner of transmission of information.

Servers 710 include one or more communication servers that are capable of communicating with storage system 750, computing device 760, and patient devices 720, 730, and 740 via network 4282. As will be described in greater detail below, servers 710 transmit subscriptions over network 4282 to patient devices 720, 730, and 740. In turn, patient devices 720, 730, and 740 transmit data to server 710 in accordance with the received subscriptions.

Computing device 760 may be configured similarly to the servers 710, with one or more processors 762, memory 764 that may comprise data and instructions as described above. Each computing device 760 may be a personal computing device intended for use by a clinician, technician, or other user and have all of the components normally used in connection with a personal computing device such as a central processing unit (CPU), memory (e.g., RAM and internal hard drives) storing data and instructions, a display such as a display 766 (e.g., a monitor having a screen, a touch-screen, a projector, a television, or other device that is operable to display information), and user input device 768 (e.g., a mouse, keyboard, touch-screen or microphone).

### 4.6 EXAMPLE METHODS

A patient device, namely RPT 4000, collects and transmits usage data in accordance with a subscription that has been stored in a memory. Usage data may include any data that relates to the patient's use of the medical device, and a subscription takes the form of a set of instructions, such as a script, that is used by the medical device in collecting and transmitting the usage data. A subscription may select the specific data that is to be collected by the patient device. For example, RPT 4000 may collect usage data relating to the duration of time that the patient has used RPT 4000, including usage data relating to the time periods in which patient interface 3000 was or was not being worn by the patient. In addition, the collected usage data may relate to how the patient is responding to the treatment. For example, the subscription being implemented by RPT 4000 may call for the collection of usage data that may be used to calculate the patient's apnea index ("AI"), hypopnea index ("HI"), or apnea-hypopnea index ("AHI"). In particular, RPT 4000 may use sensors, such as the sensors described above for measuring mass flow rate and outlet pressure, in order to track disruptions in the patient's breathing that occur over the time period for which RPT 4000 is being used. This usage data is collected and transmitted to a server in accordance with the subscription, so that a clinician may be able to review the patient's response to the therapy, including the patient's AI, HI, and AHI.

Returning to Fig. 7, patient device 720 stores one or more subscriptions 716 in memory 4260. Patient device 720 then implements a subscription 726 using central controller 4230 so as to collect and store usage data 728. Stored usage data 728 is transmitted over network 4282 to a remote device, namely server 710. A clinician may then use computing device 760 to access the transmitted usage data 718 stored at server 710. In one example, server 710 may store usage data 718 that has been transmitted by patient device 720 at a storage system 750, and computing device 760 may directly access the data stored at storage system 750.

Subscription 726 designates specific times or instances in which patient device 720 is to transmit usage data 728 that it has collected. These designated times and instances may be referred to as a triggering event. The triggering event is defined in the subscription. One such triggering event includes a predetermined time period after which the patient has stopped using patient device 720. For example, subscription 716 may indicate that usage data 728 should be transmitted one hour after a patient has stopped using patient device 720. Thus, when one hour expires since the device has been used, data will be transmitted to the server 710. If the patient stops using patient device 720, but then resumes using it within one hour, usage data 728 will not be sent. Instead, patient device 720 will wait until the patient's use of patient device 720 has stopped for the designated time period of one hour before sending usage data 728. If power to patient device 720 is switched off prior to reaching the post-treatment time period, patient device 720 may transmit usage data 728 once it enters a power-on condition, provided that the post-treatment time period has been satisfied.

In another example, the triggering event is a treatment session that has lasted a predetermined minimum time period, such as an individual treatment session that has lasted at least four hours. In still another example, the triggering event may be a combination of the four hour minimum treatment period and the one hour post-treatment period, so that usage data 728 will not be sent until both the minimum treatment period and post-treatment period are satisfied. By waiting a predetermined time period before sending usage data 728, subscription 726 may prevent unnecessary transmissions of usage data that are due to brief interruptions to treatment, such as when the patient adjusts or briefly removes patient interface 3000. The predetermined time period used to trigger the transmission of usage data 728 may be configurable for the particular subscription 726 being implemented by a specific patient device. The period of elapsed time for post-treatment or minimum treatment triggering events may be determined based on sleep data for a particular patient or for a group of patients. Accordingly, patient device 720 may implement a subscription with a predetermined time period that is different than the predetermined time period implemented by patient device 730.

In another example, subscription 726 designates a triggering event based on a cumulative amount of time that a patient has received treatment with patient device 720. This time period may also be configurable within the subscription so as to find a balance between limiting the amount of data that is transferred over network 4282 and achieving timeliness of the usage data. Subscription 726 may also cause patient device 720 to transmit zero usage data 728 or other data indicative of the case that no treatment has occurred over a predetermined time period. For example, patient device 720 may transmit usage data 728 to server 710 including zero hours/minutes of usage, indicating that patient device 720 has not been used in the last twenty-four hours. Alternatively, patient device 720 may transmit notification to server 710 indicating the lack of use of the patient device 720. Upon receiving this notification, server 710 may transmit a message to computing device 760. This message could provide information to the user of computing device 760 regarding the lack of use of patient device 720 and indicate that the patient using patient device 720 needs further support.

Subscription 726 may designate additional conditions that must be met before patient device 720 transmits usage data 728. For example, patient device may operate in any one of a plurality of therapy modes. For example, RPT 4000 may operate in a CPAP mode that is controlled by the amount of positive pressure being applied over patient interface 3000. RPT 4000 may also operate in a mode in which ventilation is provided in a non-CPAP mode. In yet another mode, the treatment may be based on the volume of air being circulated by RPT 4000. In accordance with one aspect, subscription 726 may indicate that usage data will only be transferred in connection with a particular mode of operation. For example, patient device 720 may implement subscription 726 in which usage data 728 is transmitted solely in connection with patient device 720 running in CPAP mode. Alternatively, patient device 720 may also implement a separate subscription related to transmission of usage data 728 in connection with non-CPAP modes.

In another aspect, subscription 726 instructs patient device 720 to transmit usage data 728 when a particular change in some aspect of the usage data 728 has occurred. For example, patient device 720 may track usage data 728 in accordance with subscription 726 to determine if the current usage data has changed in some way from the last time usage data 728 had been transmitted. A change in usage data 728 may include the patient's AHI, AI, or HI deviating from a predetermined range. In this way, a clinician may be able to immediately receive an indication that the patient's response to the therapy has changed. However, such "upon change" triggering is more often associated with settings that do not change frequently, such as a change in the prescription settings or a fault condition in one or more components of patient device 720. A fault condition may include any instance when one or more components of patient device 720 have not operated correctly or have been used incorrectly. Since the prescription settings changes and fault conditions are reported infrequently, such "upon change" triggering usually occurs less often than daily.

A change in usage data may also include a determination that the use of patient device 720 has deviated by more than a predetermined amount from the last time usage data was transmitted. In this way, patient device 720 may only need to transmit usage data when the patient's therapy has changed or when the patient has begun to deviate from the prescribed therapy. Typically this is applicable to usage data that changes infrequently. Examples of such usage data include changes in the therapy or comfort settings. These may be subscribed to by change such that the device only reports changes in the settings when they occur. Other examples for reporting on change are the events of hardware faults.

As set forth above, usage data 728 may be divided into different subsets. For example, subsets of usage data 728 may include device settings, usage logs, fault logs, event logs, patient conditions-such as AHI, HI, AI, respiratory rate, tidal volume, minute ventilation, and RERA data-as well as other therapy data. Subsets of usage data 728 may also include summaries of usage data versus detailed usage data. In addition, subsets of usage data may be dependent on the device type, as it may vary depending on the type of patient device being used. For example, a CPAP device may have subsets of usage data 728 that differ from the subsets used for a BiLevel PAP device. For each subset of usage data 728, subscription 726 may identify a particular triggering event or combination of triggering events that will cause patient device 720 to transmit the subset of usage data to server 710. Triggering events may include periodically timed triggers, predefined changes in the collected data or in the patient's condition, a request for the usage data from a remote device, or the occurrence of an event, such as stopping a treatment session. For example, AHI data may be transmitted based on a periodic triggering event, such as once a day, while a fault log may be transmitted from patient device 720 to server 710 as soon as a change in a fault condition of patient device 720 has been detected. In this way, the fault log data may be immediately accessible by a technician in order to potentially address the fault. In another example, usage data related to device settings or faults may be sent once a day, but only if the settings or faults for the device have changed. In another example, some subsets of usage data 728 may only be sent upon receiving a request from another device, such as server 710, for the particular subset of usage data 728. In yet another example, a summary of usage data may be sent on a regular basis, such as at the end of a treatment session, or at the end of the day if the device is not used. In contrast, detailed usage data may be sent less frequently, or only when requested. The summary usage data may be presented as one representative value that indicates a summary of the usage data during the period represented by the data. More detailed usage data may also be transmitted in some compressed format along with the summary usage data, if such detailed data needed. In this way, a patient device 720 may provide desired usage data 728, while minimising the frequency, quantity, bandwidth, and cost of the data transmissions.

Subscriptions 726 may be updated or otherwise changed in response to changing patient conditions or therapy prescriptions. For example, patient device 720 may include, upon manufacture, a first set of subscriptions 726. However, over a period of time, server 710 transmits updated or entirely new subscriptions to patient device 720. These new subscriptions are then stored by patient device 720 and implemented in accordance with the subscriptions' instructions. Alternatively, instead of one, a number of predefined subscriptions or sets of subscriptions 726 may be stored at a particular time (e.g. upon manufacture) in the patient device's memory 4260. In this case, a current subscription may still be updated by the server 710 to the respective patient device. Alternatively, a different pre-installed subscription 726 may simply be selected out of the available number of subscriptions on the patient device.

Individual data items may be selected or deselected as part of subscription 726, based on user needs. For example, individual data items may include usage data relating to usage time, AHI, HI, AI respiratory rate, tidal volume, minute ventilation, RERA, device settings, and the like. A subscription update may either subscribe to or unsubscribe from each of these data items based on a selection provided by a user. A user may make selections of individual data items using computing device 760 to access server 710 so as to select data items that will be included in particular updates 716 for patient devices 720, 730 and 740.

When a patient's therapy begins, it may be beneficial to implement a subscription 726 that collects detailed therapy data for a large set of data items, such as the data items listed above. Over time, subscription 726 may include a less detailed set of data items. This may occur by the user unsubscribing to individual data items that are no longer of interest. For example, after the patient has undergone the prescribed therapy for a period of time, such as several months, a user may unsubscribe from all therapy data except for usage data and AHI data. In addition, a change in the patient's condition may cause a user to add one or more data items by selecting individual data items that are to be included in a new subscription 726. The change to subscription 726 may also occur automatically, based on a predetermined schedule, so that the therapy data collected in accordance with the subscription changes over time. This schedule may be altered by a user based on the determined efficacy of the patient's therapy or based on changes in the patient's condition.

A clinician or technician uses computing device 760 to communicate with server 710 and to select subscriptions 716 to be transmitted to one or more patient devices in connection with a subscription update. The clinician may select subscription updates for a plurality of patient devices or for a particular patient device. For example, a subscription update may be provided specifically to patient device 720 in connection with a change in the therapy requirements for the user of patient device 720. Accordingly, clinician may select patient device 720 to receive the subscription update, while patient device 730 does not. Alternatively, a subscription update may be transmitted to a group of patient devices, such as those patient devices that are of a particular model. Accordingly, while some the examples herein refer to a particular subscription being implemented or updated on a particular patient device, such as patient device 720, it should be understood that other patient devices, such as patient devices 730 and 740, may implement and update their own set of subscriptions in a similar manner.

The subscriptions selected by the clinician are transmitted from server 710 over network 4282. The transmission of the selected subscriptions 716 to a patient device may be immediate, or may occur in connection with a predetermined event. For example, server 710 may wait to transmit selected subscriptions 716 to patient device 720 until it has been determined that patient device 720 is connected to network 4282, or until patient device 720 is in the process of sending usage data 728 to server 710. The clinician may also select a particular date on which server 710 is to transmit new subscriptions 716 to one or more of the patient devices.

In one example, patient device 720 may implement a subscription 726 that provides detailed usage data over an initial period of time, such as over the initial days or weeks of treatment. After this initial period the subscription may be automatically cancelled or automatically replaced with a new subscription. This new subscription may be designed to provide less detailed usage data, such as by providing a summary of usage data that has been collected over a week or more. Subscription 726 may also be implemented for a limited time period, such as being implemented until the patient has reached his or her insurance reimbursement criteria. For example, in order to be reimbursed by insurance, a patient may be required to use patient device 720 at least five days out of the week over the course of three months. Server 710 may compare usage data 718 that has been transmitted from patient device 720 with the patient's reimbursement criteria to determine when the criteria have been met. Once the reimbursement criteria has been met, server 710 may transmit a new subscription 716 to patient device 720 or simply cancel the current subscription 726 being implemented by patient device 720. In another example, subscription 726 may include a predetermined range of dates for which subscription 726 is to be implemented. Patient device 720 may automatically begin implementation of subscription 726 when the patient's treatment falls within the predetermined range of dates and automatically end implementation of subscription 726 when the treatment falls outside of the predetermined range of dates. For example, a subscription may target the period until re-imbursement of the newly purchased device, during which more detailed data may be required. Another example is a prescription that only targets the 2 year warrantee period, during which data is required, as in most cases no data is required after that period.

Usage data 718 stored at server 710 may be accessed by computing device 760, so that the clinician may monitor the condition of specific patients. Accordingly, usage data 718 may be stored at server 710 in a manner that associates the usage data with a particular patient device and patient. In this way, a clinician may easily detect any issues with a particular patient's therapy. Usage data 728 stored at patient devices 720, 730, and 740 may also be provided on demand to allow for immediate review. For example, server 710 or computing device 760 may request for immediate transmittal of particular usage data from patient device 720, such as all usage data 728 that was collected over a particular time period or a list of current settings for patient device 720. This may occur by server 710 transmitting a "send now" message to patient device 720 that indicates the particular set of data that is to be provided by patient device 720.

Usage data 728 may be transmitted, in accordance with the subscription, to different end points over network 4282 based on the contents or type of usage data 728 that is being transmitted. In particular, some usage data 728 may be provided directly to computing device 760 or storage system 750, while other usage data 728 may be provided to one or more servers 710. For example, a clinician may wish to directly receive usage data 728 for any patient who has experienced an unusual response to the prescribed therapy, as indicated by the patient's AHI, AI, or HI. Accordingly, subscription 726 may designate that usage data 728 having a particular AHI, AI, or HI value be sent directly to computing device 760.

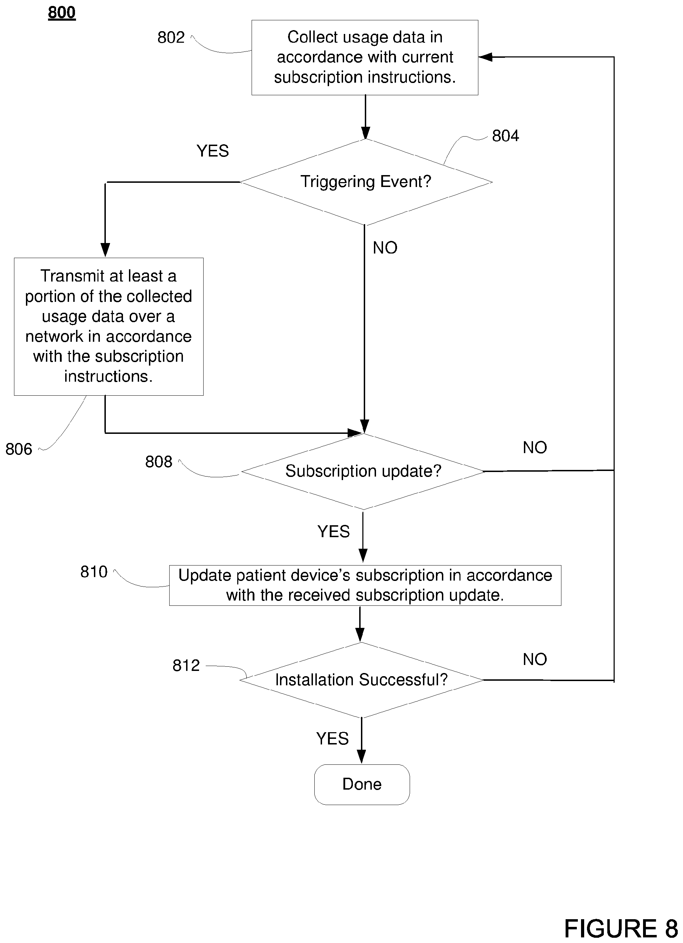

Fig. 8 is a flow diagram 800 that may be performed by a patient device of the disclosed system described above. In block 802, the patient device collects usage data in accordance with a subscription. Patient device also determines whether a triggering event has occurred (Block 804). Once a triggering event has been identified, the patient device transmits at least a portion of the collected usage data over a network, namely transmitting the usage data to a server (Block 806). As set forth above, the subscription contains the information that identifies a triggering event as well as information identifying the usage data that is to be transmitted over the network. Accordingly, the subscription may identify different subsets of data to be sent based on different triggering events. For example, a triggering event may include a determination that the patient has stopped using the patient device for a predetermined period of time, and the subscription may identify that the portion of usage data to be transmitted includes all usage data collected since the last data transmission. Another triggering event may be a change in the patient device's settings, in which the subscription could instruct the patient device to transmit a particular set of data relating to the settings of the patient device. A subscription may also identify some combination of triggering events, such as triggering events that are based on both a periodic condition and an "upon change" condition.

The patient device also determines whether a subscription update has been received (Block 808). If a subscription update has not been received, the patient device may continue to collect usage data and determine whether a triggering event has occurred in accordance with Blocks 802 and 804. However, if a subscription update has been received, the patient device may perform the update (Block 810). The update may include adding new subscriptions, altering current subscriptions, replacing current subscriptions with new subscriptions, and / or cancelling subscriptions. As described above, the subscription update may occur based on a user selecting individual data items, such as usage data and AHI data, that are to be included in a new subscription for the patient device. In addition, the subscription updates may be transmitted to the patient device in accordance with a predetermined schedule. If all subscriptions have not been cancelled (Block 812), the patient device continues to collect usage data (Block 802) and transmit usage date (Block 806) in accordance with the updated subscription instructions.

While the operations set forth in Fig. 8 may each be performed by a single patient device, some of the operations may be performed by a separate device. For example, the patient device may communicate with a personal computer over a wireless network, so that the personal computer may perform one or more of the operations described above. Operations may be added or removed from diagram 800. In addition, various operations need not be performed in the same order as set forth in diagram 800.

### 4.7 GLOSSARY

In certain forms of the present technology, one or more of the following definitions may apply. In other forms of the present technology, alternative definitions may apply.

### 4.7.1 General

Air: Air will be taken to include breathable gases, for example air with supplemental oxygen.

Continuous Positive Airway Pressure (CPAP): CPAP treatment will be taken to mean the application of a supply of air or breathable gas to the entrance to the airways at a pressure that is continuously positive with respect to atmosphere, and preferably approximately constant through a respiratory cycle of a patient. In some forms, the pressure at the entrance to the airways will vary by a few centimeters of water within a single respiratory cycle, for example being higher during inhalation and lower during exhalation. In some forms, the pressure at the entrance to the airways will be slightly higher during exhalation, and slightly lower during inhalation. In some forms, the pressure will vary between different respiratory cycles of the patient, for example being increased in response to detection of indications of partial upper airway obstruction, and decreased in the absence of indications of partial upper airway obstruction.

### 4.7.2 Materials

Silicone or Silicone Elastomer: A synthetic rubber. In this specification, a reference to silicone is a reference to liquid silicone rubber (LSR) or a compression moulded silicone rubber (CMSR). One form of commercially available LSR is SILASTIC (included in the range of products sold under this trademark), manufactured by Dow Corning. Another manufacturer of LSR is Wacker. Unless otherwise specified to the contrary, a preferred form of LSR has a Shore A (or Type A) indentation hardness in the range of about 35 to about 45 as measured using ASTM D2240.

Polycarbonate: a typically transparent thermoplastic polymer of Bisphenol-A Carbonate.

### 4.7.3 Aspects of a patient interface

Anti-asphyxia valve (AAV): The component or sub-assembly of a mask system that, by opening to atmosphere in a failsafe manner, reduces the risk of excessive CO₂ rebreathing by a patient.

Elbow: A conduit that directs an axis of flow of air to change direction through an angle. In one form, the angle may be approximately 90 degrees. In another form, the angle may be less than 90 degrees. The conduit may have an approximately circular cross-section. In another form the conduit may have an oval or rectangular cross-section.

Frame: Frame will be taken to mean a mask structure that bears the load of tension between two or more points of connection with a headgear. A mask frame may be a non-airtight load bearing structure in the mask. However, some forms of mask frame may also be air-tight.

Headgear: Headgear will be taken to mean a form of positioning and stabilizing structure designed for use on a head. Preferably the headgear comprises a collection of one or more struts, ties and stiffeners configured to locate and retain a patient interface in position on a patient's face for delivery of respiratory therapy. Some ties are formed of a soft, flexible, elastic material such as a laminated composite of foam and fabric.

Membrane: Membrane will be taken to mean a typically thin element that has, preferably, substantially no resistance to bending, but has resistance to being stretched.

Plenum chamber: a patient interface plenum chamber will be taken to mean a portion of a patient interface having walls enclosing a volume of space, such as for a full-face mask (e.g., nose and mouth mask), a nasal mask or a nasal pillow, the volume having air therein pressurised above atmospheric pressure in use by the patient. A shell may form part of the walls of a patient interface plenum chamber. In one form, a region of the patient's face abuts one of the walls of the plenum chamber, such as via a cushion or seal.

Seal: The noun form ("a seal") will be taken to mean a structure or barrier that intentionally resists the flow of air through the interface of two surfaces. The verb form ("to seal") will be taken to mean to resist a flow of air.

Shell: A shell will preferably be taken to mean a curved structure having bending, tensile and compressive stiffness, for example, a portion of a mask that forms a curved structural wall of the mask. Preferably, compared to its overall dimensions it is relatively thin. In some forms, a shell may be faceted. Preferably such walls are airtight, although in some forms they may not be airtight.

Stiffener: A stiffener will be taken to mean a structural component designed to increase the bending resistance of another component in at least one direction.

Strut: A strut will be taken to be a structural component designed to increase the compression resistance of another component in at least one direction.

Swivel: (noun) A subassembly of components configured to rotate about a common axis, preferably independently, preferably under low torque. In one form, the swivel may be constructed to rotate through an angle of at least 360 degrees. In another form, the swivel may be constructed to rotate through an angle less than 360 degrees. When used in the context of an air delivery conduit, the sub-assembly of components preferably comprises a matched pair of cylindrical conduits. Preferably there is little or no leak flow of air from the swivel in use.

Tie: A tie will be taken to be a structural component designed to resist tension.

Vent: (noun) the structure that allows a deliberate controlled rate leak of air from an interior of the mask, or conduit to ambient air, to allow washout of exhaled carbon dioxide (CO₂) and supply of oxygen (O₂).

### 4.8 OTHER REMARKS

A portion of the disclosure of this patent document contains material which is subject to copyright protection. The copyright owner has no objection to the facsimile reproduction by anyone of the patent document or the patent disclosure, as it appears in the Patent and Trademark Office patent file or records, but otherwise reserves all copyright rights whatsoever.

Unless the context clearly dictates otherwise and where a range of values is provided, it is understood that each intervening value, to the tenth of the unit of the lower limit, between the upper and lower limit of that range, and any other stated or intervening value in that stated range is encompassed within the technology. The upper and lower limits of these intervening ranges, which may be independently included in the intervening ranges, are also encompassed within the technology, subject to any specifically excluded limit in the stated range. Where the stated range includes one or both of the limits, ranges excluding either or both of those included limits are also included in the technology.

Furthermore, where a value or values are stated herein as being implemented as part of the technology, it is understood that such values may be approximated, unless otherwise stated, and such values may be utilized to any suitable significant digit to the extent that a practical technical implementation may permit or require it.

Unless defined otherwise, all technical and scientific terms used herein have the same meaning as commonly understood by one of ordinary skill in the art to which this technology belongs. Although any methods and materials similar or equivalent to those described herein can also be used in the practice or testing of the present technology, a limited number of the exemplary methods and materials are described herein.

When a particular material is identified as being preferably used to construct a component, obvious alternative materials with similar properties may be used as a substitute. Furthermore, unless specified to the contrary, any and all components herein described are understood to be capable of being manufactured and, as such, may be manufactured together or separately.

It must be noted that as used herein and in the appended claims, the singular forms "a", "an", and "the" include their plural equivalents, unless the context clearly dictates otherwise.

The publications discussed herein are provided solely for their disclosure prior to the filing date of the present application. Nothing herein is to be construed as an admission that the present technology is not entitled to antedate such publication by virtue of prior invention. Further, the dates of publication provided may be different from the actual publication dates, which may need to be independently confirmed.

Moreover, in interpreting the disclosure, all terms should be interpreted in the broadest reasonable manner consistent with the context. In particular, the terms "comprises" and "comprising" should be interpreted as referring to elements, components, or steps in a non-exclusive manner, indicating that the referenced elements, components, or steps may be present, or utilized, or combined with other elements, components, or steps that are not expressly referenced.

The subject headings used in the detailed description are included only for the ease of reference of the reader and should not be used to limit the subject matter found throughout the disclosure or the claims. The subject headings should not be used in construing the scope of the claims or the claim limitations.

Although the technology herein has been described with reference to particular embodiments, it is to be understood that these embodiments are merely illustrative of the principles and applications of the technology. In some instances, the terminology and symbols may imply specific details that are not required to practice the technology. For example, although the terms "first" and "second" may be used, unless otherwise specified, they are not intended to indicate any order but may be utilised to distinguish between distinct elements. Furthermore, although process steps in the methodologies may be described or illustrated in an order, such an ordering is not required. Those skilled in the art will recognize that such ordering may be modified and/or aspects thereof may be conducted concurrently or even synchronously.

It is therefore to be understood that numerous modifications may be made to the illustrative embodiments and that other arrangements may be devised without departing from the scope of the technology.

**LIST OF REFERENCE NUMBERS**

| | |
|---|---|
| communications system | 700 |
| server | 710 |
| processor | 712 |
| memory | 714 |
| instruction | 715 |
| subscription | 716 |
| usage data | 718 |
| patient device | 720 |
| subscription | 726 |
| usage data | 728 |
| patient device | 730 |
| patient device | 740 |
| storage system | 750 |
| computing device | 760 |
| processor | 762 |
| memory | 764 |
| display | 766 |
| user input device | 768 |
| patient | 1000 |
| patient interface | 3000 |
| seal-forming structure | 3100 |
| plenum chamber | 3200 |
| perimeter | 3210 |
| position and stabilising structure | 3300 |
| vent | 3400 |
| connection port | 3600 |
| RPT device | 4000 |
| external housing | 4010 |
| upper portion of external housing | 4012 |
| lower portion of external housing | 4014 |
| panel | 4015 |
| chassis | 4016 |
| handle | 4018 |
| pneumatic block | 4020 |
| pneumatic component | 4100 |
| air filter | 4110 |
| inlet air filter | 4112 |
| outlet air filter | 4114 |
| muffler | 4120 |
| inlet muffler | 4122 |
| outlet muffler | 4124 |
| pressure device | 4140 |
| blower | 4142 |
| motor | 4144 |
| brushless DC motor | 4144 |
| back valve | 4160 |
| air circuit | 4170 |
| supplemental oxygen | 4180 |
| electrical component | 4200 |
| board assembly PCBA | 4202 |
| power supply | 4210 |
| input device | 4220 |
| central controller | 4230 |
| clock | 4232 |
| therapy device controller | 4240 |
| protection circuit | 4250 |
| memory | 4260 |
| transducer | 4270 |
| pressure sensor | 4271 |
| .pressure transducer | 4272 |
| flow sensor | 4274 |
| motor speed signal | 4276 |
| data communication system | 4280 |
| remote external communication network | 4282 |
| local external communication network | 4284 |
| remote external device | 4286 |
| local external device | 4288 |
| output device | 4290 |
| display driver | 4292 |
| display | 4294 |
| algorithm | 4300 |
| pressure control module | 4330 |
| humidifier | 5000 |
| humidifier controller | 5250 |

## Claims

1. A system of one or more servers (710) for transmission of patient data that is associated with use of respiratory pressure therapy devices (720) by patients, each of the respiratory pressure therapy devices storing one or more subscriptions (726), each of the one or more subscriptions comprising a set of instructions configured to be used by the respiratory pressure therapy device in collecting and transmitting usage data (728) of the respiratory pressure therapy device including data that relates to the patient's use of the respiratory pressure therapy device, each of the one or more subscriptions defining at least one triggering event for causing the respiratory pressure therapy device to transmit usage data identified in the subscription, the one or more servers comprising one or more processors (712) and being configured to:
receive from a computing device (760), over a network (4282), a user selection of one or more subscriptions for one or more respiratory pressure therapy devices to receive one or more subscription updates (716);
transmit, over the network, the one or more subscription updates to the one or more of the respiratory pressure therapy devices such that the one or more respiratory pressure therapy devices can update at least one prior subscription stored on the one or more of the respiratory pressure therapy devices; and
receive, over the network, at least a portion of the usage data from one or more of the respiratory pressure therapy devices that has been collected and transmitted by the one or more of the respiratory pressure therapy devices in accordance with the updated one or more subscriptions, wherein the transmission of the usage data is caused by a determination, by the one or more of the respiratory pressure therapy devices, that a triggering event defined in the updated one or more subscriptions has occurred;
wherein the triggering event:
a) is based on a particular change in some aspect of the usage data having occurred; and/or
b) is based on a patient having finished using the respiratory pressure therapy device for a predetermined period of time; and/or
c) comprises a treatment session having lasted a predetermined minimum time period; and/or
d) is based on a cumulative amount of time that the patient has received treatment with the respiratory pressure therapy device; and/or
e) is a change in one or more settings of the respiratory pressure therapy device; and/or
f) is a fault condition in one or more components of the respiratory pressure therapy device.

2. The system of claim 1, wherein the usage data (728) is divided into different subsets comprising: (a) device settings, (b) usage logs, (c) fault logs, (d) event logs, and (e) patient conditions.

3. The system of any one of claims 1 to 2, wherein the one or more triggering events is based on a particular change in an aspect of the usage data (728) having occurred, and optionally, wherein the particular change in the aspect of the usage data comprises determining that the use of the respiratory pressure therapy device (720) has deviated by more than a predetermined amount from the last time usage data was transmitted.

4. The system of any one of claims 1 to 3, wherein the usage data (728) identifies time periods in which the respiratory pressure therapy device (720) has been used.

5. The system of any one of claims 1 to 4, wherein the usage data (728) relates to at least one of the following: a patient's apnea index, hypopnea index, apnea-hypopnea index, pressure statistics, leak statistics, minute ventilation, tidal volume and prescription settings.

6. The system of any one of claims 1 to 5, wherein the subscription (726) was generated, at least in part, based on a user's selection of individual data items that are to be collected by the respiratory pressure therapy device (720).

7. The system of any one of claims 1 to 6, wherein the one or more triggering events further include receiving a request for usage data (728).

8. The system of any one of claims 1 to 7, wherein the respiratory pressure therapy device (720) is configured so that if power to the respiratory pressure therapy device is switched off prior to a triggering event, the respiratory pressure therapy device will determine whether the triggering event has occurred upon the respiratory pressure therapy device being powered on.

9. The system of any one of claims 1 to 8, wherein the subscription (726) identifies a first subset of usage data (728) to be provided as summary usage data and a second subset of usage data to be provided as detailed usage data.

10. The system of any one of claims 1 to 9, wherein the portion of the usage data (728) that is transmitted decreases over time.

11. The system of any one of claims 1 to 10, wherein the portion of the usage data (728) that is transmitted is provided as a representative value that indicates a summary of the usage data.

12. The system of any one of claims 1 to 11, wherein the subscription (726) indicates that the portion of the usage data (728) will only be transferred in connection with a particular mode of operation.

13. The system of any one of claims 1 to 12 wherein the transmitting the selection of the one or more subscriptions (726) to the one or more of the respiratory pressure therapy devices (720) occurs in connection with a predetermined event, and optionally, wherein the predetermined event comprises one of: (1) a date selected by a clinician, (2) a determination that the one or more of the respiratory pressure therapy devices are connected to the network (4282), or (3) the one or more of the respiratory pressure therapy devices are in the process of sending usage data (728) to the one or more servers (710).

14. A method for a system of one or more servers (710) for transmission of patient data that is associated with use of respiratory pressure therapy devices (720) by patients, each of the respiratory pressure therapy devices storing one or more subscriptions (726), each of the one or more subscriptions comprising a set of instructions configured to be used by the respiratory pressure therapy device in collecting and transmitting usage data (728) of the respiratory pressure therapy device including data that relates to the patient's use of the respiratory pressure therapy device, each of the one or more subscriptions defining at least one triggering event for causing the respiratory pressure therapy device to transmit usage data identified in the subscription, the one or more servers comprising one or more processors (712), the method being carried out by the one or more servers and comprising the steps of:
receiving from a computing device (760), over a network (4282), a user selection of one or more subscriptions for one or more respiratory pressure therapy devices to receive one or more subscription updates (716);
transmitting, over the network, the one or more subscription updates to the one or more of the respiratory pressure therapy devices such that the one or more respiratory pressure therapy devices can update at least one prior subscription stored on the one or more respiratory pressure therapy devices; and
receiving, over the network, at least a portion of the usage data from one or more of the respiratory pressure therapy devices that has been collected and transmitted by the one or more of the respiratory pressure therapy devices in accordance with the updated one or more subscriptions, wherein the transmission of the usage data is caused by a determination, by the one or more of the respiratory pressure therapy devices, that a triggering event defined in the updated one or more subscriptions has occurred;
wherein the triggering event:
a) is based on a particular change in some aspect of the usage data having occurred; and/or
b) is based on a patient having finished using the respiratory pressure therapy device for a predetermined period of time; and/or
c) comprises a treatment session having lasted a predetermined minimum time period; and/or
d) is based on a cumulative amount of time that the patient has received treatment with the respiratory pressure therapy device; and/or
e) is a change in one or more settings of the respiratory pressure therapy device; and/or
f) is a fault condition in one or more components of the respiratory pressure therapy device.

## Patentansprüche

1. System mit einem oder mehreren Servern (710) zur Übertragung von Patientendaten, die mit der Verwendung von Atemdrucktherapievorrichtungen (720) durch Patienten zusammenhängen, wobei jede der Atemdrucktherapievorrichtungen ein oder mehrere Abonnements (726) speichert, wobei jedes der ein oder mehreren Abonnements einen Satz von Anweisungen aufweist, die dazu konfiguriert sind, von der Atemdrucktherapievorrichtung beim Erfassen und Übertragen von Nutzungsdaten (728) der Atemdrucktherapievorrichtung verwendet zu werden, einschließlich Daten, die sich auf die Verwendung der Atemdrucktherapievorrichtung durch den Patienten beziehen, wobei jedes der ein oder mehreren Abonnements mindestens ein auslösendes Ereignis definiert, um zu bewirken, dass die Atemdrucktherapievorrichtung in dem Abonnement identifizierte Nutzungsdaten überträgt, wobei der eine oder die mehreren Server einen oder mehrere Prozessoren (712) umfassen und dazu konfiguriert sind:
Empfangen, von einer Rechenvorrichtung (760) über ein Netzwerk (4282), einer Benutzerauswahl eines oder mehrerer Abonnements, damit eine oder mehrere Atemdrucktherapievorrichtungen eine oder mehrere Abonnementaktualisierungen (716) empfangen;
Übertragen, über das Netzwerk, der einen oder mehreren Abonnementaktualisierungen an die eine oder mehreren der Atemdrucktherapievorrichtungen, sodass die eine oder mehreren Atemdrucktherapievorrichtungen mindestens ein früheres Abonnement aktualisieren können, das auf der einen oder den mehreren der Atemdrucktherapievorrichtungen gespeichert ist; und
Empfangen, über das Netzwerk, zumindest eines Teils der Nutzungsdaten von einer oder mehreren der Atemdrucktherapievorrichtungen, die von der einen oder den mehreren der Atemdrucktherapievorrichtungen in Übereinstimmung mit dem aktualisierten einen oder den aktualisierten mehreren Abonnements erfasst und übertragen wurden, wobei die Übertragung der Nutzungsdaten durch eine Feststellung, durch die eine oder mehreren der Atemdrucktherapievorrichtungen, bewirkt wird, dass ein in dem aktualisierten einen oder den aktualisierten mehreren Abonnements definiertes auslösendes Ereignis eingetreten ist;
wobei das auslösende Ereignis:
a) auf einer eingetretenen bestimmten Änderung eines Aspekts der Nutzungsdaten basiert; und/oder
b) darauf basiert, dass ein Patient die Verwendung der Atemdrucktherapievorrichtung für eine vorbestimmte Zeitspanne beendet hat; und/oder
c) aufweist, dass eine Behandlungssitzung eine vorbestimmte Mindestzeitspanne gedauert hat; und/oder
d) auf einer kumulativen Zeitmenge basiert, in der der Patient Behandlung mit der Atemdrucktherapievorrichtung erhalten hat; und/oder
e) eine Änderung einer oder mehrerer Einstellungen der Atemdrucktherapievorrichtung ist; und/oder
f) ein Fehlerzustand in einer oder mehreren Komponenten der Atemdrucktherapievorrichtung ist.

2. System nach Anspruch 1, wobei die Nutzungsdaten (728) in unterschiedliche Teilmengen unterteilt sind, umfassend: (a) Geräteeinstellungen, (b) Nutzungsprotokolle, (c) Fehlerprotokolle, (d) Ereignisprotokolle und (e) Patientenzustände.

3. System nach einem der Ansprüche 1 bis 2, wobei das eine oder die mehreren auslösenden Ereignisse auf einer eingetretenen bestimmten Änderung eines Aspekts der Nutzungsdaten (728) basieren, und wobei optional die bestimmte Änderung des Aspekts der Nutzungsdaten das Feststellen aufweist, dass die Verwendung der Atemdrucktherapievorrichtung (720) um mehr als einen vorbestimmten Betrag von dem letzten Zeitpunkt abgewichen ist, zu dem Nutzungsdaten übertragen wurden.

4. System nach einem der Ansprüche 1 bis 3, wobei die Nutzungsdaten (728) Zeitspannen identifizieren, in denen die Atemdrucktherapievorrichtung (720) verwendet wurde.

5. System nach einem der Ansprüche 1 bis 4, wobei sich die Nutzungsdaten (728) auf zumindest eines der folgenden beziehen: Apnoe-Index, Hypopnoe-Index, Apnoe-Hypopnoe-Index , Druckstatistik, Leckstatistik, Minutenventilation, Tidalvolumen und Verordnungsparameter eines Patienten.

6. System nach einem der Ansprüche 1 bis 5, wobei das Abonnement (726) zumindest teilweise basierend auf einer Auswahl einzelner Datenelemente durch einen Benutzer erzeugt wurde, die von der Atemdrucktherapievorrichtung (720) erfasst werden sollen.

7. System nach einem der Ansprüche 1 bis 6, wobei das eine oder die mehreren auslösenden Ereignisse ferner den Empfang einer Anforderung von Nutzungsdaten (728) aufweisen.

8. System nach einem der Ansprüche 1 bis 7, wobei die Atemdrucktherapievorrichtung (720) so konfiguriert ist, dass, wenn die Stromversorgung der Atemdrucktherapievorrichtung vor einem auslösenden Ereignis ausgeschaltet wird, die Atemdrucktherapievorrichtung feststellt, ob das auslösende Ereignis eingetreten ist, wenn die Atemdrucktherapievorrichtung eingeschaltet wird.

9. System nach einem der Ansprüche 1 bis 8, wobei das Abonnement (726) eine erste Teilmenge von Nutzungsdaten (728), die als zusammenfassende Nutzungsdaten bereitzustellen ist, und eine zweite Teilmenge von Nutzungsdaten, die als detaillierte Nutzungsdaten bereitzustellen ist, identifiziert.

10. System nach einem der Ansprüche 1 bis 9, wobei der Teil der Nutzungsdaten (728), der übertragen wird, mit der Zeit abnimmt.

11. System nach einem der Ansprüche 1 bis 10, wobei der Teil der Nutzungsdaten (728), der übertragen wird, als repräsentativer Wert bereitgestellt wird, der eine Zusammenfassung der Nutzungsdaten angibt.

12. System nach einem der Ansprüche 1 bis 11, wobei das Abonnement (726) angibt, dass der Teil der Nutzungsdaten (728) nur in Verbindung mit einem bestimmten Betriebsmodus übertragen wird.

13. System nach einem der Ansprüche 1 bis 12, wobei das Übertragen der Auswahl des einen oder der mehreren Abonnements (726) an die eine oder mehreren der Atemdrucktherapievorrichtungen (720) in Verbindung mit einem vorbestimmten Ereignis erfolgt,
und wobei optional das vorbestimmte Ereignis eines aufweist aus: (1) einem von einem Kliniker ausgewählten Datum, (2) einer Feststellung, dass die eine oder mehreren der Atemdrucktherapievorrichtungen mit dem Netzwerk (4282) verbunden sind, oder (3) dass die eine oder mehreren der Atemdrucktherapievorrichtungen dabei sind, Nutzungsdaten (728) an den einen oder die mehreren Server (710) zu senden.

14. Verfahren für ein System mit einem oder mehreren Servern (710) zur Übertragung von Patientendaten, die mit der Verwendung von Atemdrucktherapievorrichtungen (720) durch Patienten zusammenhängen, wobei jede der Atemdrucktherapievorrichtungen ein oder mehrere Abonnements (726) speichert, wobei jedes der ein oder mehreren Abonnements einen Satz von Anweisungen umfasst, die dazu konfiguriert sind, von der Atemdrucktherapievorrichtung beim Erfassen und Übertragen von Nutzungsdaten (728) der Atemdrucktherapievorrichtung verwendet zu werden, einschließlich Daten, die sich auf die Verwendung der Atemdrucktherapievorrichtung durch den Patienten beziehen, wobei jedes der ein oder mehreren Abonnements mindestens ein auslösendes Ereignis definiert, um zu bewirken, dass die Atemdrucktherapievorrichtung in dem Abonnement identifizierte Nutzungsdaten überträgt, wobei der eine oder die mehreren Server einen oder mehrere Prozessoren (712) aufweisen, wobei das Verfahren durch den einen oder die mehreren Server ausgeführt wird und die Schritte aufweist:
Empfangen, von einer Rechenvorrichtung (760) über ein Netzwerk (4282), einer Benutzerauswahl eines oder mehrerer Abonnements, damit eine oder mehrere Atemdrucktherapievorrichtungen eine oder mehrere Abonnementaktualisierungen (716) empfangen;
Übertragen, über das Netzwerk, der einen oder mehreren Abonnementaktualisierungen an die eine oder mehreren der Atemdrucktherapievorrichtungen, sodass die eine oder mehreren Atemdrucktherapievorrichtungen mindestens ein früheres Abonnement aktualisieren können, das auf der einen oder den mehreren Atemdrucktherapievorrichtungen gespeichert ist; und
Empfangen, über das Netzwerk, zumindest eines Teils der Nutzungsdaten von einer oder mehreren der Atemdrucktherapievorrichtungen, die von der einen oder den mehreren der Atemdrucktherapievorrichtungen in Übereinstimmung mit dem aktualisierten einen oder den aktualisierten mehreren Abonnements erfasst und übertragen wurden, wobei die Übertragung der Nutzungsdaten durch eine Feststellung, durch die eine oder mehreren der Atemdrucktherapievorrichtungen, bewirkt wird, dass ein in dem aktualisierten einen oder den aktualisierten mehreren Abonnements definiertes auslösendes Ereignis eingetreten ist;
wobei das auslösende Ereignis:
a) auf einer eingetretenen bestimmten Änderung eines Aspekts der Nutzungsdaten basiert; und/oder
b) darauf basiert, dass ein Patient die Verwendung der Atemdrucktherapievorrichtung für eine vorbestimmte Zeitspanne beendet hat; und/oder
c) aufweist, dass eine Behandlungssitzung eine vorbestimmte Mindestzeitspanne gedauert hat; und/oder
d) auf einer kumulativen Zeitmenge basiert, in der der Patient Behandlung mit der Atemdrucktherapievorrichtung erhalten hat; und/oder
d) eine Änderung einer oder mehrerer Einstellungen der Atemdrucktherapievorrichtung ist; und/oder
e) ein Fehlerzustand in einer oder mehreren Komponenten der Atemdrucktherapievorrichtung ist.

## Revendications

1. Système d'un ou de plusieurs serveurs (710) de transmission de données de patients qui sont associées à une utilisation de dispositifs de thérapie par pression respiratoire (720) par des patients, chacun des dispositifs de thérapie par pression respiratoire maintenant un ou plusieurs abonnements (726), chaque abonnement des un ou plusieurs abonnements comprenant un ensemble d'instructions configurées pour être utilisées par le dispositif de thérapie par pression respiratoire lors d'une collecte et d'une transmission de données d'utilisation (728) du dispositif de thérapie par pression respiratoire comprenant des données qui sont associées à l'utilisation par le patient du dispositif de thérapie par pression respiratoire, chaque abonnement des un ou plusieurs abonnements définissant au moins un événement de déclenchement destiné à amener le dispositif de thérapie par pression respiratoire à transmettre des données d'utilisation identifiées dans l'abonnement, les un ou plusieurs serveurs comprenant un ou plusieurs processeurs (712) et étant configurés pour :
recevoir, d'un dispositif informatique (760), sur un réseau (4282), une sélection d'utilisateur d'un ou de plusieurs abonnements afin qu'un ou plusieurs dispositifs de thérapie par pression respiratoire reçoivent une ou plusieurs mises à jour d'abonnement (716) ;
transmettre, sur le réseau, les une ou plusieurs mises à jour d'abonnement aux un ou plusieurs dispositifs de thérapie par pression respiratoire de sorte que les un ou plusieurs dispositifs de thérapie par pression respiratoire peuvent mettre à jour au moins un abonnement antérieur maintenu par les un ou plusieurs dispositifs de thérapie par pression respiratoire ; et
recevoir, sur le réseau, au moins une partie des données d'utilisation en provenance d'un ou de plusieurs des dispositifs de thérapie par pression respiratoire qui ont été collectées et transmises par les un ou plusieurs dispositifs de thérapie par pression respiratoire conformément aux un ou plusieurs abonnements mis à jour, dans lequel la transmission des données d'utilisation est provoquée par une détermination, par les un ou plusieurs dispositifs de thérapie par pression respiratoire, de l'occurrence d'un événement de déclenchement défini dans les un ou plusieurs abonnements mis à jour ;
dans lequel l'événement de déclenchement :
a) est basé sur l'occurrence d'une modification particulière d'un certain aspect des données d'utilisation ; et/ou
b) est basé sur le fait qu'un patient a cessé d'utiliser le dispositif de thérapie par pression respiratoire pendant une période de temps prédéterminée ; et/ou
c) comprend le fait qu'une session de traitement a duré une période de temps minimale prédéterminée ; et/ou
d) est basé sur une quantité de temps cumulée durant laquelle le patient a reçu un traitement avec le dispositif de thérapie par pression respiratoire ; et/ou
e) est une modification d'un ou de plusieurs paramètres du dispositif de thérapie par pression respiratoire ; et/ou
f) est une condition de défaut d'un ou de plusieurs composants du dispositif de thérapie par pression respiratoire.

2. Système selon la revendication 1, dans lequel les données d'utilisation (728) sont divisées en différents sous-ensembles comprenant : (a) des paramètres de dispositifs, (b) des journaux d'utilisation, (c) des journaux de défauts, (d) des journaux d'événements, et (e) des conditions de patients.

3. Système selon l'une quelconque des revendications 1 à 2, dans lequel les un ou plusieurs événements de déclenchement sont basés sur l'occurrence d'une modification particulière d'un aspect des données d'utilisation (728),
et éventuellement, dans lequel la modification particulière de l'aspect des données d'utilisation comprend la détermination du fait que l'utilisation du dispositif de thérapie par pression respiratoire (720) s'est écartée de plus d'une quantité prédéterminée depuis la dernière transmission des données de utilisation.

4. Système selon l'une quelconque des revendications 1 à 3, dans lequel les données d'utilisation (728) identifient des périodes de temps pendant lesquelles le dispositif de thérapie par pression respiratoire (720) a été utilisé.

5. Système selon l'une quelconque des revendications 1 à 4, dans lequel les données d'utilisation (728) se rapportent à au moins l'un des éléments suivants : un indice d'apnée, un indice d'hypopnée, un indice d'apnée-hypopnée, des statistiques de pression, des statistiques de fuite, une ventilation par minute, un volume respiratoire et des paramètres de prescription d'un patient.

6. Système selon l'une quelconque des revendications 1 à 5, dans lequel l'abonnement (726) a été généré, au moins en partie, sur la base d'une sélection par l'utilisateur d'éléments de données individuels qui doivent être collectés par le dispositif de thérapie par pression respiratoire (720).

7. Système selon l'une quelconque des revendications 1 à 6, dans lequel les un ou plusieurs événements de déclenchement comprennent en outre la réception d'une demande de données d'utilisation (728).

8. Système selon l'une quelconque des revendications 1 à 7, dans lequel le dispositif de thérapie par pression respiratoire (720) est configuré de sorte que, si de la puissance appliquée au dispositif de thérapie par pression respiratoire est commutée hors circuit avant un événement de déclenchement, le dispositif de thérapie par pression respiratoire déterminera si l'événement de déclenchement s'est produit alors que le dispositif de thérapie par pression respiratoire était alimenté.

9. Système selon l'une quelconque des revendications 1 à 8, dans lequel l'abonnement (726) identifie un premier sous-ensemble de données d'utilisation (728) à fournir en tant que données d'utilisation résumées et un second sous-ensemble de données d'utilisation à fournir en tant que données d'utilisation détaillées.

10. Système selon l'une quelconque des revendications 1 à 9, dans lequel la partie des données d'utilisation (728) qui est transmise diminue dans le temps.

11. Système selon l'une quelconque des revendications 1 à 10, dans lequel la partie des données d'utilisation (728) qui est transmise est fournie en tant que valeur indicative qui indique un résumé des données d'utilisation.

12. Système selon l'une quelconque des revendications 1 à 11, dans lequel l'abonnement (726) indique que la partie des données d'utilisation (728) ne sera transférée qu'en lien avec un mode de fonctionnement particulier.

13. Système selon l'une quelconque des revendications 1 à 12, dans lequel la transmission de la sélection des un ou plusieurs abonnements (726) aux un ou plusieurs dispositifs de thérapie par pression respiratoire (720) se produit en lien avec un événement prédéterminé,
et éventuellement, dans lequel l'événement prédéterminé comprend l'un : (1) d'une date sélectionnée par un clinicien, (2) d'une détermination du fait que les un ou plusieurs dispositifs de thérapie par pression respiratoire sont connectés au réseau (4282), ou (3) les un ou plusieurs dispositifs de thérapie par pression respiratoire sont dans le processus d'envoi de données d'utilisation (728) aux un ou plusieurs serveurs (710).

14. Procédé destiné à un système d'un ou de plusieurs serveurs (710) de transmission de données de patients qui sont associées à une utilisation de dispositifs de thérapie par pression respiratoire (720) par des patients, chacun des dispositifs de thérapie par pression respiratoire maintenant un ou plusieurs abonnements (726), chaque abonnement des un ou plusieurs abonnements comprenant un ensemble d'instructions configurées pour être utilisées par le dispositif de thérapie par pression respiratoire lors d'une collecte et d'une transmission de données d'utilisation (728) du dispositif de thérapie par pression respiratoire comprenant des données qui sont associées à l'utilisation par le patient du dispositif de thérapie par pression respiratoire, chaque abonnement des un ou plusieurs abonnements définissant au moins un événement de déclenchement destiné à amener le dispositif de thérapie par pression respiratoire à transmettre des données d'utilisation identifiées dans l'abonnement, les un ou plusieurs serveurs comprenant un ou plusieurs processeurs (712), le procédé étant exécuté par les un ou plusieurs serveurs et comprenant les étapes consistant à :
recevoir, d'un dispositif informatique (760), sur un réseau (4282), une sélection d'utilisateur d'un ou de plusieurs abonnements afin qu'un ou plusieurs dispositifs de thérapie par pression respiratoire reçoivent une ou plusieurs mises à jour d'abonnement (716) ;
transmettre, sur le réseau, les une ou plusieurs mises à jour d'abonnement aux un ou plusieurs dispositifs de thérapie par pression respiratoire de sorte que les un ou plusieurs dispositifs de thérapie par pression respiratoire peuvent mettre à jour au moins un abonnement antérieur maintenu par les un ou plusieurs dispositifs de thérapie par pression respiratoire ; et
recevoir, sur le réseau, au moins une partie des données d'utilisation en provenance d'un ou de plusieurs des dispositifs de thérapie par pression respiratoire qui ont été collectées et transmises par les un ou plusieurs dispositifs de thérapie par pression respiratoire conformément aux un ou plusieurs abonnements mis à jour, dans lequel la transmission des données d'utilisation est provoquée par une détermination, par les un ou plusieurs dispositifs de thérapie par pression respiratoire, de l'occurrence d'un événement de déclenchement défini dans les un ou plusieurs abonnements mis à jour ;
dans lequel l'événement de déclenchement :
a) est basé sur l'occurrence d'une modification particulière d'un certain aspect des données d'utilisation ; et/ou
b) est basé sur le fait qu'un patient a cessé d'utiliser le dispositif de thérapie par pression respiratoire pendant une période de temps prédéterminée ; et/ou
c) comprend le fait qu'une session de traitement a duré une période de temps minimale prédéterminée ; et/ou
d) est basé sur une quantité de temps cumulée durant laquelle le patient a reçu un traitement avec le dispositif de thérapie par pression respiratoire ; et/ou
e) est une modification d'un ou de plusieurs paramètres du dispositif de thérapie par pression respiratoire ; et/ou
f) est une condition de défaut d'un ou de plusieurs composants du dispositif de thérapie par pression respiratoire.
